# DEMANDE DE BREVET EUROPEEN

(11) **EP 1 308 501 A1**
(43) Date de publication de la demande: **07.05.2003**
(21) Numéro de dépôt: 02292724.8
(22) Date de dépôt: 31.10.2002
(51) Int. Cl.: C12C 11/00, C12G 1/02, C12G 3/02, C12N 1/18, C12N 15/63

(54) **Boissons fermentées comprenant des levures transformees par le gene ATF2**

(30) Priorité: 02.11.2001 FR 0114221
(71) Demandeur: Pernod-Ricard, 75016 Paris (FR)
(72) Inventeur: Degryse, Eric, 91560 Crosne (FR); Girard, Patrick, 91940 Les Ulis (FR); Godard, Nicolas, 75020 Paris (FR)
(74) Mandataire: Ahner, Francis

(57) **Abrégé**

La présente invention concerne l'utilisation d'une composition de microorganismes transformés par le gène *ATF2,* pour la préparation d'une boisson fermentée enrichie en esters acétylés, caractérisée en ce que la concentration de la boisson fermentée en esters acétylés indésirables d'un point de vue sensoriel - plus spécialement en acétate d'éthyle - est comprise entre 10 et 150 mg/l.

LISTE DE SEQUENCES

<110> Pernod Ricard

<120> Boissons fermentées gene ATF 2

<130> D19536

<160> 2

<170> PatentIn Ver. 2.1

<210> 1
<211> 2336
<212> ADN
<213> Saccharomyces cerevisiae

<220>
<223> Alcool acetyltransferase

<400> 1
cttcagatag aacaggcttt agcaaatttt aagtaatcag tatttcaatc gccacaatct 60
caggctacat tgaactctgt aggccaccga taaatattgc gggataatga gtaaacgaat 120
tcaacgagtt tcctattttc ctattctata ttttttcatg cacctaagcg atcgtaaaca 180
tttgttgtca agatttacgt ttcttcgagg tctgtgaaaa tagagcaaat tgttacttaa 240
aagacaacaa cgaatgtgta gggcaattct gcagtagcag cactgtcatt cctaatgcgt 300
acaaacagta ctgagacttt caaacgaata ataacttcag caataaaaat tgtccaggtt 360
aattatggaa gatatagaag gatacgaacc acatatcact caagagttga tagaccgtgg 420
ccatgcaaga cgtatgggcc acttggaaaa ctactttgct gttttgagta ggcagaaaat 480
gtactcgaat tttactgttt acgcggaatt gaataaaggt gttaataaga gacaactaat 540
gcttgtcttg aaactattac ttcaaaaata ctcaactctt gcgcatacaa tcattcctaa 600
gcattatcct catcatgaag cgtactactc tagcgaagag taccttagta aacctttttcc 660
acagcatgat ttcataaagg tgatttctca tcttgaattc gatgacttga ttatgaataa 720
tcaaccagaa tacagagaag tcatggagaa aatctcagaa cagttcaaaa aggatgattt 780
caaagtcacc aataggttaa tcgaattgat tagccctgta atcatacctg tgggtaatcc 840
gaagaggcct aattggagat tgatttgttt accaggtaag gatactgatg ggtttgaaac 900
gtggaaaaac ttcgtttatg tcactaacca ctgcggctcc gacggtgtca gtggatcgaa 960
ttttttcaaa gatttagctc tactcttttg taaaatcgaa gaaaaagggt ttgattatga 1020
tgaagagttc atcgaagatc aggtcatcat tgactatgat cgagactaca ctgaaatttc 1080
taaattgcca aaaccgatta cggatcgtat tgactacaag ccagcattga cttcattacc 1140
caaattcttt ttaacaacct tcatttatga acattgtaat tttaaaacct ccagcgaatc 1200
tacacttaca gctagatata gcccctctac taatgctaat gctagttaca attacttgtt 1260
gcatttcagt actaaggaag tagaacaaat cagagcccag atcaagaaaa atgttcacga 1320
tgggtgcacc ctaacaccct tcattcaagc gtgctttctt gtagccctgt atagactaga 1380
taagttgttc acaaaatctc ttctcgagta tgggttcgat gtggctattc caagcaacgc 1440
aagaaggttt ttaccaaacg atgaagagtt aagagattct tataaatacg gctccaacgt 1500
tggaggttcg cattacgcct atctaatctc ctcattcgac attcccgaag gtgacaatga 1560
caagttttgg agtcttgtcg aatactacta tgaccgcttt ttagaatcgt acgacaacgg 1620
tgaccacttg attggtctgg gggtcctaca acttgatttt atcgttcaaa acaagaatat 1680
agacagcctt cttgccaact cttatttgca ccagcaaaga ggcggtgcaa tcatcagtaa 1740
tacaggactt gtctcgcaag atacgaccaa gccgtactac gttcgggatt taatcttctc 1800
gcagtctgca ggcgccttga gatttgcgtt cggcctaaac gtttgctcca caaacgtgaa 1860
tggtatgaac atggacatga gcgtggttca gggcactcta cgggatcgtg gcgaatggga 1920
atcgttctgc aagctcttct accaaaccat cggcgaattt gcgtcgcttt aatgcgatac 1980
tgccgtagcg ggccttcgta tagctcggcg gacgtcgtac aaaaggcaag cagtgtatcg 2040
gacagagctg atataacaca atacgctcgt agtcgatgca tgccgtggct gctctcggtc 2100
gggtataagt ctcagacaat agtcttacct cgcatgtata ataaatcttt tgtatttaat 2160
ctattatatg tttctatgct tttttttcct attgttgttt gcttttcctt ttccttattt 2220
ctttctagct tctaattttc tttcttttt tttttttttc attgaaaatt atatatatat 2280
atatatatca gaacaattgt ccagtattga acaatacagg ttatttcgaa caattg 2336

<210> 2
<211> 535
<212> PRT
<213> Saccharomyces cerevisiae

<220>
<223> Alcool acetyltransferase

<400> 2
Met Glu Asp Ile Glu Gly Tyr Glu Pro His Ile Thr Gln Glu Leu Ile
1               5                   10                  15

Asp Arg Gly His Ala Arg Arg Met Gly His Leu Glu Asn Tyr Phe Ala
            20                  25                  30

Val Leu Ser Arg Gln Lys Met Tyr Ser Asn Phe Thr Val Tyr Ala Glu
        35                  40                  45

Leu Asn Lys Gly Val Asn Lys Arg Gln Leu Met Leu Val Leu Lys Val
    50                  55                  60

Leu Leu Gln Lys Tyr Ser Thr Leu Ala His Thr Ile Ile Pro Lys His
65                  70                  75                  80

Tyr Pro His His Glu Ala Tyr Tyr Ser Ser Glu Glu Tyr Leu Ser Lys
                85                  90                  95

Pro Phe Pro Gln His Asp Phe Ile Lys Val Ile Ser His Leu Glu Phe
            100                 105                 110

Asp Asp Leu Ile Met Asn Asn Gln Pro Glu Tyr Arg Glu Val Met Glu
        115                 120                 125

Lys Ile Ser Glu Gln Phe Lys Lys Asp Asp Phe Lys Val Thr Asn Arg
    130                 135                 140

Leu Ile Glu Leu Ile Ser Pro Val Ile Ile Pro Leu Gly Asn Pro Lys
145                 150                 155                 160

Arg Pro Asn Trp Arg Leu Ile Cys Leu Pro Gly Lys Asp Thr Asp Gly
                165                 170                 175

Phe Glu Thr Trp Lys Asn Phe Val Tyr Val Thr Asn His Cys Gly Ser
            180                 185                 190

Asp Gly Val Ser Gly Ser Asn Phe Phe Lys Asp Leu Ala Leu Leu Phe
        195                 200                 205

Cys Lys Ile Glu Glu Lys Gly Phe Asp Tyr Asp Glu Glu Phe Ile Glu
    210                 215                 220

Asp Gln Val Ile Ile Asp Tyr Asp Arg Asp Tyr Thr Glu Ile Ser Lys
225                 230                 235                 240

Leu Pro Lys Pro Ile Thr Asp Arg Ile Asp Tyr Lys Pro Ala Leu Thr
                245                 250                 255

Ser Leu Pro Lys Phe Phe Leu Thr Thr Phe Ile Tyr Glu His Cys Asn
            260                 265                 270

Phe Lys Thr Ser Ser Glu Ser Thr Leu Thr Ala Arg Tyr Ser Pro Ser
        275                 280                 285

Ser Asn Ala Asn Ala Ser Tyr Asn Tyr Leu Leu His Phe Ser Thr Lys
    290                 295                 300

Gln Val Glu Gln Ile Arg Ala Gln Ile Lys Lys Asn Val His Asp Gly
305                 310                 315                 320

Cys Thr Leu Thr Pro Phe Ile Gln Ala Cys Phe Leu Val Ala Leu Tyr
                325                 330                 335

Arg Leu Asp Lys Leu Phe Thr Lys Ser Leu Leu Glu Tyr Gly Phe Asp
            340                 345                 350

Val Ala Ile Pro Ser Asn Ala Arg Arg Phe Leu Pro Asn Asp Glu Glu
        355                 360                 365

Leu Arg Asp Ser Tyr Lys Tyr Gly Ser Asn Val Gly Gly Ser His Tyr
    370                 375                 380

Ala Tyr Leu Ile Ser Ser Phe Asp Ile Pro Glu Gly Asp Asn Asp Lys
385                 390                 395                 400

Phe Trp Ser Leu Val Glu Tyr Tyr Tyr Asp Arg Phe Leu Glu Ser Tyr
                405                 410                 415

Asp Asn Gly Asp His Leu Ile Gly Leu Gly Val Leu Gln Leu Asp Phe
            420                 425                 430

Ile Val Glu Asn Lys Asn Ile Asp Ser Leu Leu Ala Asn Ser Tyr Leu
        435                 440                 445

His Gln Gln Arg Gly Gly Ala Ile Ile Ser Asn Thr Gly Leu Val Ser
    450                 455                 460

Gln Asp Thr Thr Lys Pro Tyr Tyr Val Arg Asp Leu Ile Phe Ser Gln
465                 470                 475                 480

Ser Ala Gly Ala Leu Arg Phe Ala Phe Gly Leu Asn Val Cys Ser Thr
                485                 490                 495

Asn Val Asn Gly Met Asn Met Asp Met Ser Val Val Gln Gly Thr Leu
            500                 505                 510

Arg Asp Arg Gly Glu Trp Glu Ser Phe Cys Lys Leu Phe Tyr Gln Thr
        515                 520                 525

Ile Gly Glu Phe Ala Ser Leu
    530                 535

## Description

L'invention concerne notamment l'utilisation d'une composition de microorganismes transformés par le gène *ATF2*, pour la préparation d'une boisson fermentée enrichie en esters acétylés sans surproduction d'esters acétylés indésirables, un procédé de préparation d'une telle boisson, une boisson obtenue par ce procédé.

L'appréciation sensorielle globale des boissons fermentées fait intervenir des composés aromatiques spécifiques, notamment les esters acétylés. En l'absence de composés malodorants, une variété immense de substances chimiques développe un arôme agréable, associé à la spécificité du produit qui se distingue dans sa classe (par exemple du vin muscaté en comparaison du Riesling). Malgré le fait que certaines familles de composés soient associées à certaines boissons (les terpènes et le vin muscaté par exemple), de manière générale, un seul composé dominant n'est pas à l'origine de l'arôme d'un produit fini. En effet, un arôme de produit fermenté résulte plutôt de proportions équilibrées d'une panoplie de composés. Mais les composés aromatiques ne seront pas perçus si leur concentration chute en dessous du seuil de perception et à l'inverse si leur concentration devient trop élevée ces mêmes composés peuvent devenir néfastes au goût et à l'arôme du produit fini.

La formation d'un milieu odorant par la levure dépend des conditions de fermentation ainsi que de la libération d'un certain nombre de facteurs variétaux (propres au cépage) particuliers : par exemple, sous l'activité d'une β-glucosidase, les terpènes du vin muscaté peuvent être libérés. La levure contribue donc à préparer des changements aromatiques plus complexes lors du vieillissement (par exemple: extraction des arômes du chêne - frits, copeaux...).

Mis à part l'éthanol, la levure génère, durant la fermentation, un certain nombre de métabolites secondaires qui contribuent à la valeur sensorielle du produit fermenté. Ces composés aromatiques sont produits par la levure, dans des rapports différents, et dépendent de nombreuses variables, telles la température, la matière première et la souche utilisée. La levure crée donc un environnement aromatique en produisant des alcools supérieurs, des esters acétylés et éthylés. On recherche une meilleure connaissance des facteurs qui régissent la synthèse de ces composés aromatiques pour améliorer le procédé de préparation de la boisson fermentée et du produit fini.

Les esters acétylés sont formés à partir des alcools correspondants. Il existe par exemple des techniques qui permettent de sélectionner des souches de levure qui ont une synthèse accrue de l'isoamylalcool ou du 2-phényléthanol. (Casalone *et al.,* 1997 ; Cavalieri *et al.,* 1999 ; Fukuda *et al*., 1991 a, b). Il n'en résulte pas pour autant une augmentation de la teneur en esters acétylés. Ainsi une modulation de la production des esters acétylés peut être envisagée par une surexpression du ou des gènes impliqués dans cette production, ou par une connaissance de leur régulation.

La synthèse d'esters d'acétate a été entreprise d'un point de vue biochimique par Malcorps et Dufour (1992) qui ont démontré la présence d'une activité alcool acétyle transférase « induite » à la fin de la phase exponentielle (absence d'oxygène). Cette induction est « réduite » par l'ajout d'acide linoléique. L'enzyme partiellement purifié est actif sur l'éthanol, l'isoamylalcool et le 12-DL-hydroxystéarate. Il utilise comme co-facteur l'acétyle-CoA, mais pas l'hexanoyle-CoA. Alors que le Km pour l'acétyle-CoA est relativement bas (25-40 µM), celui pour l'accepteur est élevé : 1M pour l'éthanol, 25mM pour l'isoamylalcool.

Le gène *ATF1* qui code pour une protéine de 525 acides aminés a été isolé à partir de *S. cerevisae* (Fuji *et al*., 1994). Le gène *ATF1* se trouve dans *S. cerevisae* et des souches vinicoles. *S. carlsbergensis* contient une copie additionnelle, *Lg-ATF1*, qui code pour une protéine très similaire de 545 acides aminés (Fuji *et al*., 1996). L'influence de l'extension N-terminale de 20 acides aminés sur la fonction de l'enzyme n'est pas connue.

Des études ont révélé la régulation de l'expression du gène codant pour une alcool acétyltransférase.

L'influence de l'oxygène et des acides gras non saturés a été étudiée. L'expression du gène *ATF1* a été suivie par dosage de son messager. Le gène sous contrôle de son promoteur naturel subit une répression forte en présence d'oxygène, donc en conditions aérobies (Fuki *et al*., 1997).

Un deuxième niveau de régulation opère en conditions anaérobies. En effet, comme déjà noté plus haut (Malcorps et Dufour, 1992), l'expression du gène subit une répression par des acides gras non saturés. L'effet étant le plus important pour l'acide linoléique (18:2) et linolénique (18:3) suivi de l'acide oléique (18 :1) alors que l'acide stéarique (18 :0) n'exerce aucune influence. Lorsque les cultures sont transférées de conditions anaérobies, soit en aérobie, soit en anaérobie mais avec l'ajout d'acide oléique, *ATF1* est réprimé complètement en 30 minutes. Par contre, lorsque l'on ajoute de l'ergostérol aucun effet n'est mesurable (Fuji *et al*., 1997 ; Fujiwara *et al*., 1998, 1999). Donc, l'oxygène, les acides gras non saturés, mais pas les stérols, répriment l'expression d'*ATF1.*

L'influence d'une activité estérase a été en outre mise en évidence. Toutefois, le produit de l'activité de la protéine, principalement l'acétate d'isoamyle, est soumis à une hydrolyse enzymatique (Fuji *et al*., 1996). Une activité estérase sur l'acétate d'isoamyle a pu être mise en évidence. Le gène codant pour cette activité (*IAH1*) a été cloné et des mutants délétés ont été générés. Comparée à une souche sauvage, l'augmentation en acétate d'isoamyle est de l'ordre de 1,2 fois pour le mutant. L'influence de la mutation est particulièrement prononcée dans des souches qui surexpriment *ATF1* par le biais d'un plasmide. Dans ce cas, des augmentions d'un facteur 1.7, 3.9 et 2 ont été observées pour le mutant, comparé à la souche sauvage pour respectivement l'acétate d'éthyle, l'acétate d'isobutyle et l'acétate d'isoamyle (Fukuda *et al*., 1998).

On connaît déjà des procédés de préparation de boissons fermentées mettant en oeuvre une surexpression d'*ATF1* (brevets US 5 728 412).

Avec pour but l'amélioration de la qualité aromatique du vin, en particulier lorsque le jus de raisin lui même est pauvre en précurseurs aromatiques (leucine et phénylalanine par exemple), l'effet de la surexpression d'*ATF1* sous contrôle du promoteur pro*PGK1* à partir d'une insertion génomique dans des souches oenologiques, a été étudié (Lilly, Lambrechts et Pretorius, 2000). L'influence de cette expression a été suivie par l'analyse de molécules responsables du bouquet fruité du Chenin blanc : l'acétate d'isoamyle (banane), l'acétate de 2-phényléthyle (fruité, fleuri - voir le brevet US 5 965 780), l'acétate d'hexyle (fruité), le caproate d'éthyle et le caprylate d'éthyle (pomme). La fermentation du Chenin blanc par des souches surexprimant *ATF1* montre un profil modifié pour les métabolites secondaires. La concentration d'acétate d'éthyle (vernis), d'acétate d'isoamyle et d'acétate de 2-phényléthyle augmente (de l'ordre de 2,4 - 12 fois), parallèlement à une réduction des alcools supérieurs correspondants. Par contre, les taux de méthanol et d'acide acétique sont réduits, alors que le caprate d'éthyle, le caproate d'éthyle et l'acétate d'hexyle restent inchangés. Six mois après la mise en bouteille, l'acétate d'éthyle est présent à des taux nettement moindres, mais reste de manière significative supérieur au taux de la souche commerciale non modifiée. La réduction des esters acétylés (l'acétate de 2-phényléthyle et l'acétate d'isoamyle) n'est que de l'ordre de 2 fois (Lilly, Lambrechts et Pretorius, 2000). Il semble donc que ce composé soit plus stable chimiquement et apporte plus longtemps sa contribution aromatique à la boisson fermentée.

Après distillation de vins de Colombar, le premier distillat se retrouve avec une composition similaire à celle du vin fermenté, les métabolites étant présents à plus fortes doses. Une seconde distillation élimine certains produits (l'acide acétique et autres acides) et contient des taux élevés d'esters éthylés (Lilly, Lambrechts et Pretorius, 2000).

Des changements sont perceptibles tant au niveau des vins que des produits distillés. Cette étude montre qu'un taux plus élevé de l'expression d'*ATF1* peut avoir une incidence sur le profil aromatique de boissons alcoolisées, fermentées ou distillées (Lilly, Lambrechts et Pretorius, 2000). Le problème est la surproduction de l'acétate d'éthyle, ce qui se retrouve de manière significative dans un test sensoriel (Lilly, Lambrechts et Pretorius, 2000). L'avantage d'une surexpresion d'*ATF1* est à minimiser étant donné l'accumulation d'acétate d'éthyle qui ajoute une note de vernis, souvent peu appréciée.

L'identification du gène *ATF1* a permis de générer des mutants *atf1* (Fuji *et al.,* 1996). Il a été montré qu'un mutant *atf1* est toujours capable de former 20% d'acétate d'isoamyle et plus de 60% d'acétate d'éthyle (Fuji *et al.,* 1996).

Par ailleurs, un deuxième gène, *ATF2*, a été identifié dans le génome de la levure ; il code pour une protéine, Atf2p, qui présente 37% d'identité au niveau de la séquence en acides aminés avec Atf1p. Par surexpression du gène *ATF2* à partir d'un plasmide multicopie, il a été montré que la levure transformée ne produisait que 2,4 fois plus d'acétate d'isoamyle (Nagasawa *et al*., 1998). Nagasawa *et al*. (1998) ne font pas mention d'une production d'autres dérivés acétylés tels l'acétate d'éthyle ou l'acétate de 2-phényléthyle. L'ensemble de leur étude ne permet pas de conclure si *ATF2* est responsable de l'activité résiduelle d'alcool acétyle-transférase dans une souche *atf1*, et ne décrit ni ne suggère d'une manière suffisante pour l'homme du métier l'influence du gène *ATF2* sur d'autres esters que l'acétate d'isoamyle, la mise en évidence d'une telle influence nécessitant les travaux spécifiques et complexes qui seront décrits plus loin.

Par contre, le produit du gène *ATF2* a été identifié comme étant une enzyme capable d'estérifier la pregnénolone (Cauet *et al*., 1999). Atf2p a comme substrat l'acétyl-CoA et comme accepteur d'acétyle la fonction 3β-OH des stéroïdes (mais pas des stérols). Les valeurs des Km pour l'acétyle-CoA et la pregnénolone sont très basses : 1 µM et 0.5µM respectivement, mais le turnover est faible et fut estimé à 125/min. Le rôle de l'enzyme a été étudié dans des mutants *atf2*, dont la croissance n'est pas affectée en absence de pregnénolone.

De l'ensemble des données physiologiques de l'art antérieur, il a été en outre conclu qu'au moins une fonction du gène *ATF2* est de détoxifier des métabolites pouvant affecter la croissance de la levure. Toutefois les mécanismes exacts et le contrôle de l'activité du gène *ATF2* sont encore très mal connus en comparaison de l'activité du gène *ATF1.*

L'invention vise à pallier les inconvénients de l'art antérieur et à proposer un procédé de préparation de boissons fermentées avec un profil aromatique enrichi et/ou amélioré. En particulier l'invention vise à réduire la production d'acétate d'éthyle en comparaison avec des souches qui surexpriment *ATF1.*

L'invention vise également à identifier des composés pouvant être acétylés par ATF2p pour mieux contrôler la production des esters correspondants et améliorer le profil aromatique de la boisson fermentée.

A cet effet l'invention a pour objet selon un premier aspect l'utilisation d'une composition de microorganismes transformés par le gène *ATF2*, de manière à préparer une boisson fermentée enrichie en esters acétylés apportant une note fruitée souhaitée, sans pour autant augmenter significativement la teneur en esters indésirables d'un point de vue sensoriel au delà de leur seuil de perception et notamment en acétate d'éthyle. Typiquement le seuil de perception pour l'acétate d'éthyle est de l'ordre de 50 à 150 mg/l selon le type de boisson fermentée. Cette fourchette est indicative et correspond aux principales boissons fermentées. On précise que la teneur en esters indésirables d'un point de vue sensoriel ne dépasse pas le seuil de perception connu de l'homme de l'art pour la dégustation d'une boisson fermentée donnée. Ce seuil de perception est susceptible de varier en fonction de la complexité aromatique de la boisson notamment. Toutefois il est connu sans effort excessif par l'homme du métier.

En particulier, selon une réalisation, la concentration de la boisson fermentée en esters acétylés indésirables d'un point de vue sensoriel - plus spécialement en acétate d'éthyle - est inférieure à 150mg/l, et typiquement de 10 à 50 mg/l.

Selon une réalisation, la concentration de la boisson fermentée en esters acétylés indésirables d'un point de vue sensoriel - plus spécialement en acétate d'éthyle - est de l'ordre de 3 à 30 fois inférieure à celle obtenue avec une composition de microorganismes transformés par le gène *ATF1*, et reste en dessous du seuil de perception.

Par composition de microorganismes transformés par le gène *ATF2*, en particulier de levures, on désigne une préparation solide ou liquide, comprenant essentiellement ces microorganismes obtenus après culture dans un milieu approprié, telle qu'un surnageant de culture, un isolat ou un concentrat, l'essentiel de ces microorganismes (typiquement au moins 80%) intégrant le gène *ATF2.*

Par gène *ATF2* on entend un acide nucléique codant pour l'enzyme ATF2p. On pourra utiliser en particulier la séquence ADN décrite par Nagasawa et al. (1998-Biosc.Biotechnol.Biochem.62), correspondant à SEQ ID N°1 et codant pour l'enzyme ATF2p correspondant à SEQ ID N°2. On entend également :
- a) une séquence nucléotidique comportant au moins 80 %, 85 %, 90 %, 95 % ou 98 % d'identité avec SEQ ID N° 1
- b) une séquence nucléotidique hybridant dans des conditions de forte stringence avec SEQ ID N° 1
- c) une séquence nucléotidique complémentaire de SEQ ID N° 1 ou complémentaire d'une séquence nucléotidique telle que définie en a), ou b),
- d) une séquence nucléotidique de fragment représentatif de SEQ ID N° 1, ou de fragment représentatif d'une séquence nucléotidique telle que définie en a), b) ou c);
- e) une séquence nucléotidique comprenant une séquence telle que définie en a), b), c) ou d) ;
- f) une séquence nucléotidique modifiée d'une séquence nucléotidique telle que définie en a), b), c), d) ou e).

Par acide nucléique, séquence nucléique ou d'acide nucléique, polynucléotide, oligonucléotide, séquence de polynucléotide, séquence nucléotidique, termes qui seront employés indifféremment dans la présente description, on entend désigner un enchaînement précis de nucléotides, modifiés ou non, permettant de définir un fragment ou une région d'un acide nucléique, comportant ou non des nucléotides non naturels, et pouvant correspondre aussi bien à un ADN double brin, un ADN simple brin que des produits de transcription desdits ADNs.

Ces séquences ont été prélevées directement ou indirectement, par exemple par copie, leur environnement ayant été au moins partiellement modifié. On entend ainsi également désigner les acides nucléiques obtenus par synthèse chimique.

Par « pourcentage d'identité » entre deux séquences d'acides nucléiques ou d'acides aminés au sens de la présente invention, on entend désigner un pourcentage de nucléotides ou de résidus d'acides aminés identiques entre les deux séquences à comparer, obtenu après le meilleur alignement, ce pourcentage étant purement statistique et les différences entre les deux séquences étant réparties au hasard et sur toute leur longueur. On entend désigner par "meilleur alignement" ou "alignement optimal", l'alignement pour lequel le pourcentage d'identité déterminé comme ci-après est le plus élevé. Les comparaisons de séquences entre deux séquences d'acides nucléiques ou d'acides aminés sont traditionnellement réalisées en comparant ces séquences après les avoir alignées de manière optimale, ladite comparaison étant réalisée par segment ou par « fenêtre de comparaison » pour identifier et comparer les régions locales de similarité de séquence. L'alignement optimal des séquences pour la comparaison peut être réalisé, outre manuellement, au moyen de l'algorithme d'homologie locale de Smith et Waterman (1981, Ad. App. Math. 2 : 482), au moyen de l'algorithme d'homologie locale de Neddleman et Wunsch (1970, J. Mol. Biol. 48 : 443), au moyen de la méthode de recherche de similarité de Pearson et Lipman (1988, Proc. Natl. Acad. Sci. USA 85 : 2444), au moyen de logiciels informatiques utilisant ces algorithmes (GAP, BESTFIT, BLAST P, BLAST N, FASTA et TFASTA dans le Wisconsin Genetics Software Package, Genetics Computer Group, 575 Science Dr., Madison, WI). Afin d'obtenir l'alignement optimal, on utilise de préférence le programme BLAST, avec la matrice BLOSUM 62. On peut également utiliser les matrices PAM ou PAM250.

Le pourcentage d'identité entre deux séquences d'acides nucléiques ou d'acides aminés est déterminé en comparant ces deux séquences alignées de manière optimale dans laquelle la séquence d'acides nucléiques ou d'acides aminés à comparer peut comprendre des additions ou des délétions par rapport à la séquence de référence pour un alignement optimal entre ces deux séquences. Le pourcentage d'identité est calculé en déterminant le nombre de positions identiques pour lesquelles le nucléotide ou le résidu d'acide aminé est identique entre les deux séquences, en divisant ce nombre de positions identiques par le nombre total de positions comparées et en multipliant le résultat obtenu par 100 pour obtenir le pourcentage d'identité entre ces deux séquences.

Par séquences nucléiques présentant un pourcentage d'identité d'au moins 80 %, de préférence 85 % ou 90 %, de façon plus préférée 95 % voire 98 %, après alignement optimal avec une séquence de référence, on entend désigner les séquences nucléiques présentant, par rapport à la séquence nucléique de référence, certaines modifications comme en particulier une délétion, une troncation, un allongement, une fusion chimérique et/ou une substitution, notamment ponctuelle, et dont la séquence nucléique présente au moins 80 %, de préférence 85 %, 90 %, 95 % ou 98 %, d'identité après alignement optimal avec la séquence nucléique de référence. Il s'agit de préférence de séquences dont les séquences complémentaires sont susceptibles de s'hybrider spécifiquement avec les séquences de référence. De préférence, les conditions d'hybridation spécifiques ou de forte stringence seront telles qu'elles assurent au moins 80 %, de préférence 85 %, 90 %, 95 % ou 98 % d'identité après alignement optimal entre l'une des deux séquences et la séquence complémentaire de l'autre.

Une hybridation dans des conditions de forte stringence signifie que les conditions de température et de force ionique sont choisies de telle manière qu'elles permettent le maintien de l'hybridation entre deux fragments d'ADN complémentaires. A titre illustratif, des conditions de forte stringence de l'étape d'hybridation aux fins de définir les fragments polynucléotidiques décrits ci-dessus, sont avantageusement les suivantes.

L'hybridation ADN-ADN ou ADN-ARN est réalisée en deux étapes : (1) préhybridation à 42°C pendant 3 heures en tampon phosphate (20 mM, pH 7,5) contenant 5 x SSC (1 x SSC correspond à une solution 0,15 M NaCl + 0,015 M citrate de sodium), 50 % de formamide, 7 % de sodium dodécyl sulfate (SDS), 10 x Denhardt's, 5 % de dextran sulfate et 1 % d'ADN de sperme de saumon ; (2) hybridation proprement dite pendant 20 heures à une température dépendant de la taille de la sonde (i.e. : 42°C, pour une sonde de taille > 100 nucléotides) suivie de 2 lavages de 20 minutes à 20°C en 2 x SSC + 2 % SDS, 1 lavage de 20 minutes à 20°C en 0,1 x SSC + 0,1 % SDS. Le dernier lavage est pratiqué en 0,1 x SSC + 0,1 % SDS pendant 30 minutes à 60°C pour une sonde de taille > 100 nucléotides. Les conditions d'hybridation de forte stringence décrites ci-dessus pour un polynucléotide de taille définie, peuvent être adaptées par l'homme du métier pour des oligonucléotides de taille plus grande ou plus petite, selon l'enseignement de Sambrook et al., (1989, Molecular cloning : a laboratory manual. 2^{nd} Ed. Cold Spring Harbor).

Par fragment représentatif, on entend désigner une séquence nucléique codant pour un fragment biologiquement actif d'un polypeptide ATF2p tel que défini plus loin.

Par le terme séquences contenant des séquences, ou des fragments représentatifs, on entend des séquences qui sont naturellement encadrées par des séquences qui présentent au moins 80 %, 85 %, 90 %, 95 % ou 98 % d'identité avec les séquences codant pour ATF2p.

Par séquence nucléotidique modifiée, on entend toute séquence nucléotidique obtenue par mutagénèse ou analogue selon des techniques appropriées, et comportant des modifications par rapport aux séquences normales, par exemple des mutations dans les séquences régulatrices et/ou promotrices de l'expression du polypeptide, notamment conduisant à une modification du taux d'expression ou de l'activité dudit polypeptide. Dans la présente description, les termes polypeptides, séquences polypeptidiques, peptides et protéines ATF2p sont interchangeables. Il est entendu que ces polypeptides ou fragments de polypeptides ont tous l'activité acétyl transférase Il de l'ATF2p vis-à-vis des précurseurs métaboliques cités dans la description, ce qui inclut notamment :
a) le polypeptide ATF2p de séquence SEQ ID N°2 ;
b) un polypeptide présentant au moins 70 % de préférence 80, 85 %, 90 %, 95 % et 98 % d'identité avec un polypeptide selon l'invention ;
c) un fragment biologiquement actif d'un polypeptide tel que défini en a) ou b);
d) un polypeptide modifié d'un polypeptide tel que défini en a), b), ou c).

Par polypeptide présentant un certain pourcentage d'identité avec un autre, que l'on désignera également par polypeptide homologue, on entend désigner les polypeptides présentant par rapport aux polypeptides naturels, certaines modifications, en particulier une délétion, addition ou substitution d'au moins un acide aminé, une troncation, un allongement, une solution chimérique et/ou une mutation, ou les polypeptides présentant des modifications post-traductionnelles. Parmi les polypeptides homologues, on préfère ceux dont la séquence d'acides aminés présentent au moins 80 %, de préférence 85 %, 90 %, 95 % et 98 % d'homologie avec les séquences d'acides aminés des polypeptides selon l'invention. Dans le cas d'une substitution, un ou plusieurs acide(s) aminé(s) consécutif(s) ou non consécutif(s) sont remplacés par des acides aminés « équivalents ». L'expression « acides aminés équivalents » vise ici à désigner tout acide aminé susceptible d'être substitué à l'un des acides aminés de la structure de base sans cependant modifier essentiellement les activités biologiques des peptides correspondant et telles qu'elles seront définies par la suite.

Ces acides aminés équivalents peuvent être déterminés soit en s'appuyant sur leur homologie de structure avec les acides aminés auxquels ils se substituent, soit sur des résultats d'essais comparatifs d'activité biologique entre les différents polypeptides susceptibles d'être effectués.

A titre d'exemple, on mentionne les possibilités de substitution susceptibles d'être effectuées sans qu'il résulte en une modification approfondie de l'activité biologique du polypeptide modifié correspondant. On peut remplacer ainsi la leucine par la valine ou l'isoleucine, l'acide aspartique par l'acide glutamine, la glutamine par l'asparagine, l'arginine par la lysine, etc... les substitutions inverses étant naturellement envisageables dans les mêmes conditions.

Les polypeptides homologues correspondent également aux polypeptides codés par les séquences nucléotidiques homologues ou identiques, telles que définies précédemment et comprennent ainsi dans la présente définition des polypeptides mutés et qui correspondent notamment à des troncatures, substitutions, délétions et/ou additions, d'au moins un résidu d'acides aminés.

Il est entendu que l'on calcule le pourcentage d'identité entre deux polypeptides de la même façon qu'entre deux séquences d'acides nucléiques.

Par « polypeptide modifié» on entend désigner un polypeptide ATF2p obtenu par recombinaison génétique ou par synthèse chimique, qui présente au moins une modification par rapport à la séquence normale. Ces modifications peuvent être notamment portées sur des acides aminés nécessaires pour la spécificité ou l'efficacité de l'activité, ou à l'origine de la conformation structurale, de la charge, ou de l'hydrophobicité du polypeptide selon l'invention. On peut ainsi obtenir des polypeptides dans lesquels plusieurs acides aminés peuvent être modifiés, tronqués à l'extrémité N ou C-terrninale, ou bien délétés, ou ajoutés.

Par ailleurs, de manière préférée la concentration en acétate d'éthyle de la boisson fermentée de l'ordre de 1 à 10 fois supérieure à celle obtenue avec une composition contrôle de microorganismes non transformés, et reste inférieure au seuil de perception.

La concentration de la boisson fermentée en acétate de 2-phényléthyle est de l'ordre de 7 à 70 fois supérieure à celle obtenue avec la composition contrôle, et 1.1 à 4 fois inférieure à celle obtenue avec la composition transformée par *A TF1.*

La concentration de la boisson fermentée en acétate d'isoamyle est de l'ordre de 4 à 30 fois supérieure à celle obtenue avec la composition contrôle, et 2 à 10 fois inférieure à celle obtenue avec la composition transformée par *A TF1.*

Le rapport acétate d'éthyle /acétate de 2-phényléthyle de la boisson fermentée est de l'ordre de 1.4 à 10 fois inférieur à celui d'une boisson fermentée obtenue en utilisant une composition de microorganismes transformés par le gène *A TF1.*

En particulier l'invention concerne un telle utilisation par notamment pour la préparation de vin, de bière, de cidre, de saké. Toutefois la base fermentaire peut être de n'importe quelle origine permettant d'aboutir à une boisson fermentée.

Selon un autre aspect l'invention concerne un procédé de préparation d'une boisson fermentée comprenant les étapes:
- préparation d'une base fermentaire comprenant au moins un précurseur métabolique d'ester acétylé,
- préparation d'une composition de microorganismes transformés par *ATF2,*
- addition de la composition de microorganismes transformés à la base fermentaire pour obtenir par fermentation une boisson fermentée;
les précurseurs étant choisis parmi les alcools supérieurs, les alcools primaires, les alcools secondaires, les diols, les acides aminés précurseurs des alcools correspondants, les terpènes ou tout autre substrat pour ATF2p, la concentration d'esters acétylés indésirables, plus spécialement d'acétate d'éthyle, dans la boisson fermentée étant typiquement de l'ordre de 10 à 150 mg/l, et de préférence inférieure à 50 mg/l. Cette concentration est de l'ordre de 3 à 30 fois inférieure à celle obtenue avec une composition de microorganismes transformés par le gène *A TF1.*

Selon une réalisation les alcools sont choisis parmi le n-propanol, le n-butanol, le n-hexanol, le n-heptanol, le n-octanol, le n-décanol, l'isobutanol, le 2-phényléthanol, le benzylalcool, le glycérol.

Selon une réalisation les terpènes sont choisis parmi le géraniol, le citronellol, le nérol.

Les microorganismes transformés seront typiquement des levures en particulier du genre *Saccharomyces* en particulier *S.cerevisiae et S.carlsbergensis.*
Selon une réalisation les microorganismes sont transformés par une cassette d'expression contenant le gène *A TF2* et au moins une séquence régulatrice de son expressIon.

Selon une variante la cassette d'expression est intégrée dans le génome des microorganismes à l'aide d'un vecteur intégratif

Selon une variante la cassette d'expression est introduite dans un vecteur épisomal.

Les molécules extrachromosomiques étant susceptibles d'être perdus lors d'une propagation non sélective, on pourra préférer des plasmides intégratifs, auxquels on pourra intégrer des promoteurs constitutifs forts éventuellement inductibles.

On pourra utiliser comme séquences d'initiation de la transcription des promoteurs de levures qui sont des séquences ADN régulatrices capables de lier une ADN polymérase de levure et d'initier la transcription de la séquence codante *ATF2*, notamment:
- des promoteurs associés au métabolisme de la levure tels que ceux de l'alcool déshydrogénase 1 et I1, la phosphoglucoisomérase, la glucose-6-phosphate déshydrogénase, la phosphoglycérate kinase, la phosphofructokinase, la glycéraldéhyde3-phosphate déshydrogénase, ou des séquences hybrides de ces promoteurs
- des promoteurs pour la transcription de facteurs d'initiation, de facteurs d'élongation (par exemple EF-1 et EF-2).

En particulier plusieurs promoteurs liés à la glycolyse sont activés en présence de sucre ou d'éthanol, donc lors de la fermentation.

L'avantage de l'utilisation d'un promoteur de levure est que les mécanismes propres de régulation du gène *ATF2* peuvent être contournés et le taux d'expression peut être dosé selon une vaste gamme (Nacken, Achstetter et Degryse, 1996) de sorte à obtenir une boisson fermentée où la proportion des esters acétylés apporte une amélioration en ajoutant une note agréable.

Une cellule hôte de levure transformée comprend typiquement de l'ordre de 1 à 500 copies de la cassette d'expression, selon que cette cassette est intégrée au génome ou constitue un élément extrachromosomique en plusieurs copies. La cassette d'expression est fabriquée selon des techniques connues de l'homme du métier. Les plasmides contenant la cassette sont par exemple obtenus après clonage dans *E.coli.* Les vecteurs de clonage comprennent notamment une origine de réplication fonctionnelle dans l'hôte de clonage, un marqueur de sélection de l'hôte contenant le vecteur. On peut en outre utiliser des vecteurs navettes qui comprennent au moins deux origines de réplication qui permettent au vecteur d'être répliqué à la fois dans un hôte levure pour l'expression et un hôte bactérie pour le clonage. Comme exemple de vecteurs navettes on peut citer le Yep24 (Bolstein et al. 1979 Gene 8 :17-24), pC1/1 (Brake et al. 1984, Proc.Natl.Acad.Sci.USA 1 :4642-4646). Des exemples de vecteurs intégratifs sont également décrits dans Bolstein et al. 1979 Gene 8 : 17-24, avec typiquement moins de 50 copies de la cassette d'expression intégrée dans le génome, et de préférence 1 à 10.

On peut par exemple utiliser les vecteurs Y Rp, Y Ep, Y Cp, y Ip connus de l'homme du métier. Parmi les levures, on utilisera de préférence des levures de la famille des *Saccharomycetaceae*, en particulier de la sous-famille des *Saccharomycoideae*, en particulier du genre *Saccharomyces,* et préférentiellement *Saccharomyces cerevisiae.* Par ailleurs, le gène *A TF2* décrit ici est isolé à partir de *Saccharomyces cerevisae.* Dans la présente invention on couvre toute autre source biologique permettant d'obtenir une séquence homologue à plus de 70%, en utilisant des techniques de génie génétique. Les techniques de transformation de telles levures, sont connues, par exemple de Biochemistry and Genetics of Yeast (M.Bacila, B.L.Hoerecker&AOM Stoppani eds.1978), et The Molecular Biology of the Yeast *Saccharomyces* (Strathern et al.eds.1981 ).

Une surexpression du gène *ATF2* peut être obtenue à partir de mutations induites par rayons UV ou toute autre technique permettant d'obtenir une synthèse accrue de la protéine Atf2p.

Selon un autre aspect l'invention concerne un procédé de préparation d'une boisson à partir d'une boisson fermentée telle que décrite précédemment, la boisson fermentée étant distillée et/ou servant dans un mélange de boissons fermentées ou non fermentées.

Selon un autre aspect l'invention concerne une boisson susceptible d'être obtenue selon un procédé tel que décrit précédemment.

Selon un autre aspect l'invention concerne une préparation de microorganismes transformés par le gène *ATF2*, du type surnageant de culture ou analogue, destinée à la préparation d'une telle boisson. Selon une réalisation, on procède aux étapes suivantes: transformation des levures par un plasmide comprenant *ATF2*, développement des levures, addition de ces levures à un jus de raisin, collecte du surnageant à partir du jus de raisin après la fermentation.

Selon un autre aspect l'invention concerne une souche de levure transformée destinée à une boisson fermentée transformée par le gène *A TF2,* surproductrice d'esters acétylés, sous une forme utilisable pour une industrie alimentaire, par exemple lyophilisée.

Selon un autre mode de réalisation de l'invention, on utilise une souche à terpène capable de produire au moins du géraniol et/ou de l'acétyl géraniol grâce à *ATF2,* ce qui permet d'augmenter de manière souhaitée la complexité aromatique de la boisson fermentée.

Par ailleurs, selon un mode de réalisation, on peut associer les gènes *ATF2* et *ATF1* pour ajuster le profil aromatique aux propriétés aromatiques recherchées, par une combinaison :
- d'une surexpression de *ATF1*, sous contrôle d'un promoteur faible, à partir d'une insertion génomique dans des souches oenologiques, pour augmenter la production globale d'esters aromatiques ;
- d'une surexpression de *ATF2*, pour orienter le métabolisme en limitant la production d'esters indésirables.

L'invention sera particulièrement bien comprise à l'aide de la description détaillée qui suit, en référence aux dessins annexés dans lesquels :
- la figure 1 représente le profil des métabolites volatils issus de la fermentation des souches dans un contexte génétique de mutation du gène codant pour l'acétate d'isoamyle, *iah1*(fermentation sur malt glucosé)
- la figure 2 représente la production d'esters dans les conditions de la figure 1, avec addition d'isoamylalccol dans le milieu de culture (fermentation sur malt glucosé+ isoamylalcool)
- la figure 3 représente la production d'esters dans les conditions de la figure 1, avec addition de 2-phényléthanol dans le milieu de culture (fermentation sur malt glucosé+ 2-phényléthanol)
- la figure 4 représente une carte de restriction du vecteur pRC102 de levure
- la figure 5 représente une carte de restriction du vecteur pRC103 de levure
- la figure 6 représente une carte de restriction du vecteur pRC104 de levure qui correspond à l'insertion du terminateur ter*PGK1* dans pRC103
- la figure 7 représente une carte de restriction du vecteur pRC105 de levure qui correspond à l'insertion du promoteur pro*TEF1* dans pRC104
- la figure 8 représente une carte de restriction du vecteur pRC124 de levure qui correspond à l'insertion de *ATF2* dans pRC105
- la figure 9 représente une carte de restriction du vecteur pRC131 de levure qui correspond à l'insertion de *ATF1* dans *pRC*105
- les figures 10a et 10b représentent l'effet de la surexpression respectivement du gène *ATF1* et *ATF2* sur la production d'esters
- la figure 11 représente une carte de restriction du vecteur pRC165 dans lequel le marqueur *URA3* a été remplacé par *kanR*
- la figure 12 représente une carte de restriction du vecteur pRC172 de levure qui contient la cassette d'expression du gène *ATF2* sous contrôle du promoteur pro*TEF1* et du terminateur ter*PGK1*
- la figure 13 représente la production d'esters par des souches vinicoles surexprimant les gènes *ATF1* et *ATF2*
- les figures 14 à 16 représentent des cartes de restriction des vecteurs *pRC180 à pRC184.*

On décrit ci-après de manière détaillée plusieurs modes de réalisation de l'invention. Les souches surexprimant *ATF2* ont été déposé à la CNCM sous les numéros d'enregistrement I-2739 et I-2741. Les souches surexprimant *ATF1* ont été déposées à la CNCM sous les numéros d'enregistrement I-2740 et I-2742.

### Exemple 1. Etude in vivo de l'activité alcool acétyle transférase exprimée à partir des gènes ATF1 et ATF2. Comparaison du profil de production d'esters par Saccharomyces cerevisiae dans un contexte de mutation dans le gène codant pour l'estérase de l'acétate d'isoamyle, iah1.

### Outils :

Les souches suivantes, dont les inactivations de gène ont été réalisées dans un environnement BY4740 ou BY4741, ont été obtenues auprès de Research Genetics :
4807 (*MATa, his3-Δ0, leu2-Δ0, met15-Δ0, ura3-Δ0, atf2::kanR*)
11674 (*MATα, his3-Δ0, leu2-Δ0, lys2-Δ0, ura3-Δ0, atf1::kanR*)
12382 (*MATα, his3-Δ0, leu2-Δ0, lys2-Δ0, ura3-Δ0, iah1::kanR*). Cette souche sera appelée ci-après YRC889, afin d'uniformiser la nomenclature.

Pour la construction d'un double mutant *atf2, iah1*, les inventeurs ont croisé la souche 4807 par la souche 12382 qui, après sporulation, a généré YRC988 (*MATa, his3-Δ0, leu2-Δ0, lys2-Δ0, ura3-Δ0, atf2::kanR, iah1::kanR*). A ensuite été croisée 11674 par YRC988. Des spores issues de ce croisement ont donné un double mutant YRC1018 (*MATα, his3-Δ0, leu2-Δ0, lys2-Δ0, ura3-Δ0, atf1::kanR, iah1::kanR*) et un triple mutant YRC1001 (*MATa, his3-Δ0, leu2-Δ0, lys2-Δ0, ura3-Δ0, atf1::kanR, atf2::kanR, iah1::kanR*).

### Fermentation sur malt :

Des précultures sont ensemencées pour la nuit, à 29°C, par les souches à étudier, dans du milieu gélosé YPD20 (Sherman, 1991; contenant du glucose à 2% w/v) additionné de généticine (G418) à 200 µg/ml. Les cultures ont lieu sans agitation : elles sont ensemencées à 10⁶ cellules/ml dans 150 ml de milieu malt en fioles Erlenmeyer de 150 ml. L'inoculum est constitué d'une riche suspension de cellules fraîches prélevées sur la boîte de préculture. Le milieu malt est obtenu à partir d'un malt concentré (Scotmalt, extrait de Malt liquide standard, distribué par Stern-France) dilué dans de l'eau déminéralisée afin d'obtenir une densité de 1,065. Le malt est glucosé par addition de glucose 10% w/v. Ce milieu est stérilisé pendant la nuit par ajout de 150 µl de diéthyle pyrocarbonate (DEPC, Sigma). Les exigences nutritionnelles sont compensées par supplémentation à raison d'au moins 20 µg/ml (leucine, lysine, histidine et uracile).

Le suivi de la fermentation se fait par une mesure du poids de la fiole au cours du temps (72 heures). Les pertes de poids durant la fermentation sont quasi identiques pour toutes les souches. La perte de poids d'une culture non ensemencée est inférieure à 7 % de celle d'une culture ensemencée et est donc considérée comme négligeable.

### Dosage des alcools supérieurs et leurs esters .

Afin d'établir un profil métabolique on centrifuge le milieu fermenté pendant 10 minutes à 4°C dans une centrifugeuse Jouan CR4-12 à 2000 g et on récupère le surnageant.

Afin d'identifier et de quantifier les autres composés volatils tels les alcools supérieurs et leurs esters acétylés, les acides gras et leurs esters éthylés, on extrait 20 ml du milieu centrifugé, auxquels sont ajoutés 50 µl de standard interne (pentanol-1). Après passage sur colonne ChemElut CE1020, les métabolites captés par la colonne sont ensuite élués par 3 volumes (de 20ml) de dichlorométhane. L'éluat est transféré dans un ballon surmonté d'une colonne Kuderna-Danish. Il est concentré par évaporation du solvant à l'air, en bain-marie thermostaté à 95°C, jusqu'à réduction du volume à 1ml. Les échantillons ont été analysés par chromatographie en phase gazeuse sur un appareil Agilent 6890. L'analyse se fait par le protocole suivant : Colonne FFAP (Agilent) 50 m par 320 µm de diamètre interne (pour 0,5 µm d' épaisseur du film) ; programme de température : 50°C pendant 3 minutes, puis 3°C/minute jusqu'à 120°C, 2°C/minute jusqu'à 225°C et maintenu à 225°C pendant 1 heure. Les températures du détecteur et de l'injecteur sont maintenues à 250°C. L'injection de 1 µl se fait en mode « Splitless » pendant 30 secondes ; le débit de l'hélium dans la colonne est constant à 2 ml/minute. Les produits sont détectés à l'aide d'un détecteur à ionisation de flamme. La quantité d'une substance donnée est calculée relative à l'aire du standard interne (n-pentanol).

Pour plus de commodité, l'isoamylalcool sera désigné par « IAA », le 2-phényléthanol par « FE », l'acétate d'isoamyle par « IAAc » et l'acétate de 2-phényléthyle par « FEAc »

### Résultats.

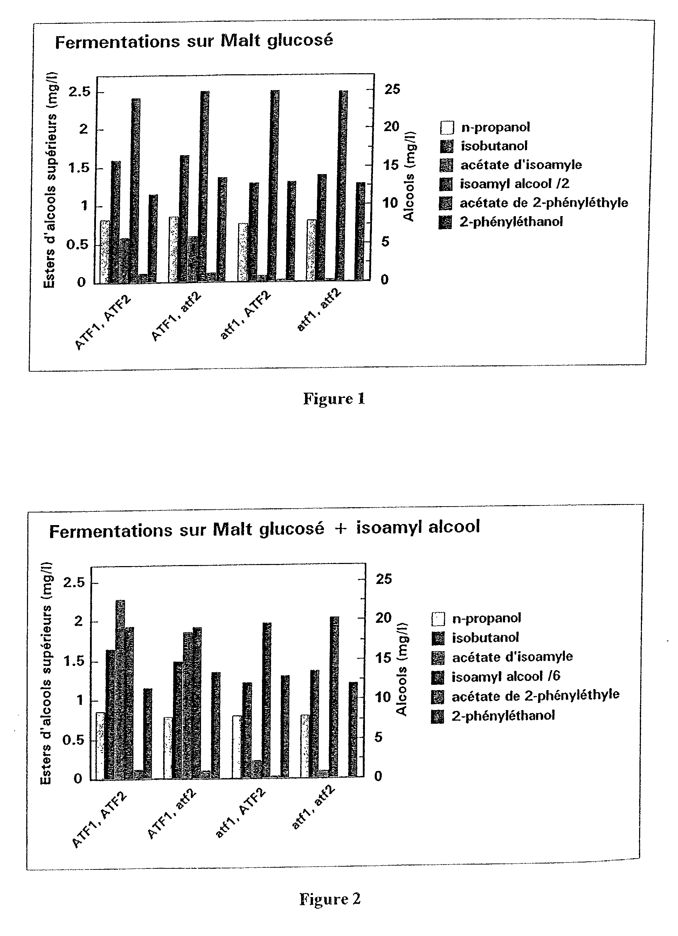

Dans la figure 1 est représenté le profil des métabolites volatils issus de la fermentation des souches dans un contexte génétique *iah1.* L'accumulation des esters acétylés d'alcools supérieurs est moins importante pour la souche YRC1018 (*ATF2*, *atf1, iah1*) que pour la souche YRC988 (*ATF1, atf2, iah1*). Ceci indique que l'activité de la protéine produite à partir du gène *ATF2* est inférieure à celle de la protéine exprimée à partir du gène *ATF1.* Mais Atf2p est aussi capable de produire des esters acétylés. Le triple mutant (*atf1, atf2, iah1*) ne produit plus qu'une très faible quantité d'esters d'alcools supérieurs.

Le rapport « acétate de 2-phényléthyle / acétate d'isoamyle » comparé à la souche sauvage, diminue dans le cas de la souche *ATF1, atf2*, passant de 0.21 à 0,186 (Tableau 1). Ceci montre une estérification plus favorable pour l'isoamylalcool que pour le 2-phényléthanol. Le même rapport passe de 0,21 à 0,41 dans le cas de la souche *atf1, ATF2* (Tableau 1). Ceci indique que la souche *atf1, ATF2* est relativement plus active sur le 2-phényléthanol.

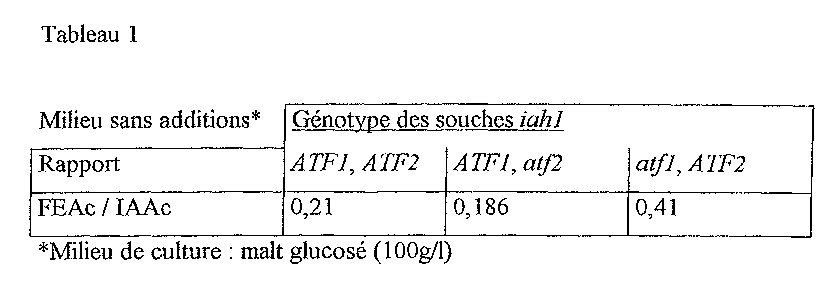

Les inventeurs ont démontré qu'Atf2p produit des esters ayant un pouvoir aromatique. Atf2p pourrait présenter un avantage si toutefois on confirme que sa spécificité est différente de celle d'Atflp. L'activité réduite d'Atf2p comparativement à Atf1p pourrait présenter un avantage dans un bilan sensoriel où l'on ne recherche pas forcément une surproduction de métabolites aromatiques mais plutôt un bon équilibre.

### Exemple 2. Etude in vivo de l'activité alcool acétyle transférase exprimée à partir des gènes ATF1 et ATF2 dans un milieu additionné d'alcools supérieurs. Comparaison du profil de production d'esters par Saccharomyces cerevisiae dans un contexte de mutation dans le gène codant pour l'estérase de l'acétate d'isoamyle, iah1.

Les matériels et méthodes sont semblables à ceux décrits dans l'exemple 1, à l'exception du milieu de culture auquel est ajouté, à raison de 100 mg/l, soit de l'isoamylalcool soit du 2-phényléthanol. Les pertes de poids durant la fermentation sont quasi identiques à celles observées sans addition (comme dans l'exemple 1), indiquant que l'ajout d'alcools supérieurs n'altère pas de manière substantielle la fermentation en anaérobie. Après 72 heures de fermentation on mesure le profil métabolique comme précédemment (exemple 1).

### Résultats :

Dans la figure 2 on représente la production d'esters lorsque l'isoamylalcool a été ajouté au milieu de culture. La quantité d'isoamylalcool extraite du milieu de fermentation est augmentée, se situant en moyenne à 118 mg/l, ce qui est à comparer au 49,5 mg/l du milieu fermenté sans addition. On s'aperçoit que la teneur en ester correspondant à l'alcool ajouté (acétate d'isoamyle) est augmentée de manière substantielle, plus pour la souche *ATF1, atf2* que pour la souche *atf1, ATF2* alors que la production de l'ester dérivé du 2-phényl éthanol (acétate de 2-phényléthyle) reste équivalente à celle obtenue dans le milieu sans addition. Le triple mutant ne produit que très peu d'esters acétylés.

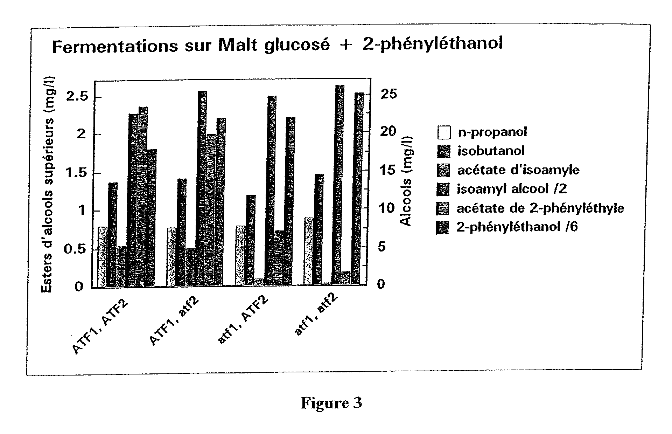

Dans la figure 3 on représente la production d'esters lorsque le 2-phényléthanol a été ajouté au milieu de culture. La quantité de 2-phényléthanol extraite du milieu de fermentation est augmentée et se situe en moyenne à 131 mg/l, à comparer avec une moyenne de 12,5 mg/l sans ajout. Le triple mutant *atf1, atf2, iah1* accumule très peu d'esters acétylés. On s'aperçoit là encore que la teneur en ester correspondant à l'alcool ajouté (acétate de 2-phényléthyle) est augmentée de manière substantielle. Ces augmentations des teneurs en esters correspondant aux alcools ajoutés indiquent une bioconversion plus importante pour la souche YRC988 (*ATF1, atf2, iah1*) que pour la souche YRC1018 (*atf1, ATF2, iah1*).

Les calculs des rapports ester formé/alcool supérieur obtenus pour les 2 milieux supplémentés en alcools supérieurs sont représentés dans le Tableau 2A et 2B. On voit que le rapport pour la souche sauvage (*ATF1, ATF2*) équivaut à la somme des rapports obtenus pour chacun des mutants [(*ATF1, atf2*) *+* (*atf1, ATF2*)], quel que soit l'ajout en alcool effectué.

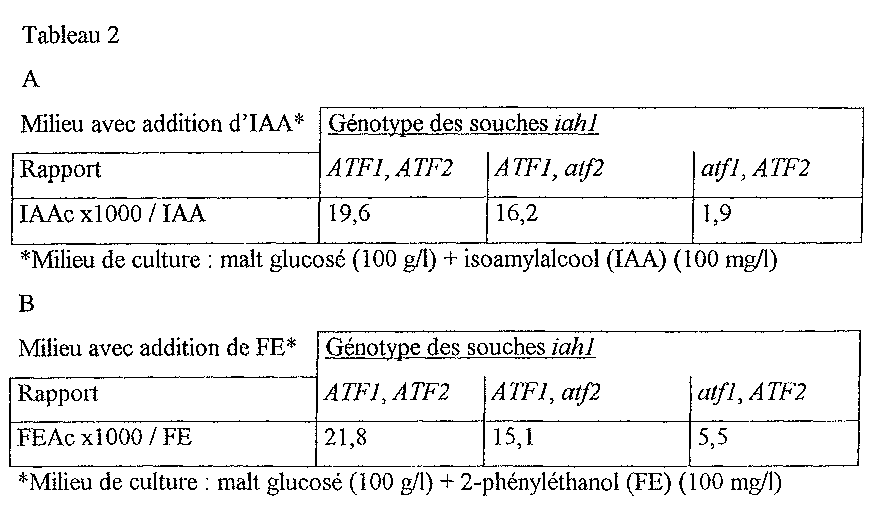

Si l'on considère les productions de l'ester correspondant à l'alcool ajouté, des différences apparaissent clairement entre les souches *ATF1, atf2* et *atf1, ATF2.* En effet, les rapports « IAAc x1000 / IAA » dans le milieu malt additionné d'isoamylalcool et « FEAc x1000 / FE » dans le milieu malt additionné de 2-phényléthanol passent de 16,2 à 15,1 pour la souche *ATF1, atf2* soit une baisse de 7% et de 1,9 à 5,5 pour la souche *atf1, ATF2* soit une augmentation de 190%. Ceci confirme une préférence de la souche *atf1, ATF2* pour le 2-phényléthanol. Donc le gène *ATF2* favorise la production de l'ester acétate de 2-phényléthyle, qui apporte des notes fruitées, fleuries, rosées, miel.

### Exemple 3 : comparaison du spectre de production d'esters à partir d'une surexpression des gènes ATF1 ou ATF2 dans un contexte génétique iah1.

### Outils :

Les gènes *ATF1* et *ATF2* ont été clonés dans des vecteurs navettes à nombre moyen de copies (2µ) avec *URA3* comme marqueur de sélection. Les plasmides pRC124 et pRC131 contiennent ainsi les cassettes d'expression des gènes *ATF2* et *ATF1* respectivement, sous contrôle du promoteur de *TEF1* (*proTEF1*) et du terminateur de *PGK1* (ter*PGK1*).

### Construction des plasmides

Les oligonucléotides suivants ont été utilisés : 
ORC 129 (ATF1 FW)
5'GTATGTCGACAACAATGAATGAAATCGATGAGAAAAA3'
ORC 130 (ATF1 RV)
5'CCAGACGCGTCAGTCAAGCATCATGTGAGATC3'
ORC 139 (ATF2 FW)
5'AAAAGTCGACAACAAATGGAAGATATAGAAGGATACGAACCAC3'
ORC 140 (ATF2 RV)
5'AAAAACGCGTAACAATTAAAGCGACGCAAATTCGCC3'
ORC 103 (proTEF1 FW)
5'AAAAGGATCCCACACACCATAGCTTCAAAATGTTTCTAC3'
 
ORC 104 (proTEF1 RV)
5'AAAAGTCGACTAGATTAGATTGCTATGCTTTC3'
ORC 107 (terPGK1 FW)
5'AAAAACGCGTCCCATGTCTCTACTGGTGGTGG3'
ORC 108 (terPGK1 RV)
5'AAAACCTAGGTTTCGAAACGCAGAATTTTCG3'
ORC 99 (Linker FW)
5'GGCCGCAAGGATCCAAGTCGACAAGAGCTCAAACGCGTAACCTAGGAAG
CGGCCGCACATG3'
ORC 100 (Linker RV)
5'TGCGGCCGCTTCCTAGGTTACGCGTTTGAGCTCTTGTCGACTTGGATCCTT
GC3'
 note: les préfixes « pro » et « ter » indiquent qu'il s'agit d'un promoteur et d'un terminateur, respectivement.

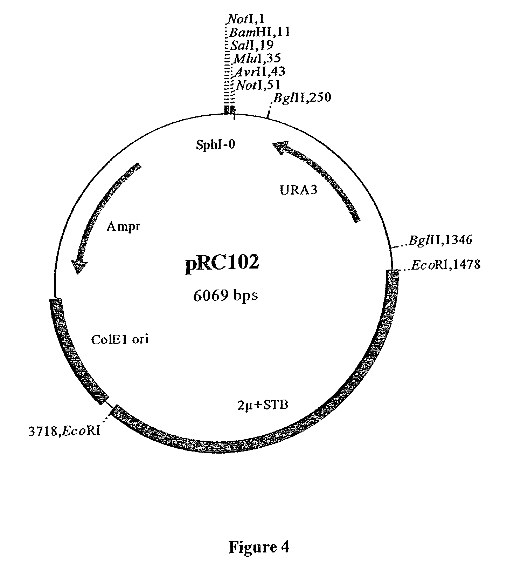

Le plasmide pRC102 correspond au plasmide pFL44L (Bonneaud *et al*., 1991) dont le site multiple de clonage (SMC) a été modifié. Le nouveau SMC, construit par hybridation des oligonucléotides ORC99 et ORC100, a été cloné dans le pFL44L préalablement digéré par *NotI* et *SphI.* Le produit de cette ligation est le pRC102 (Figure 4).

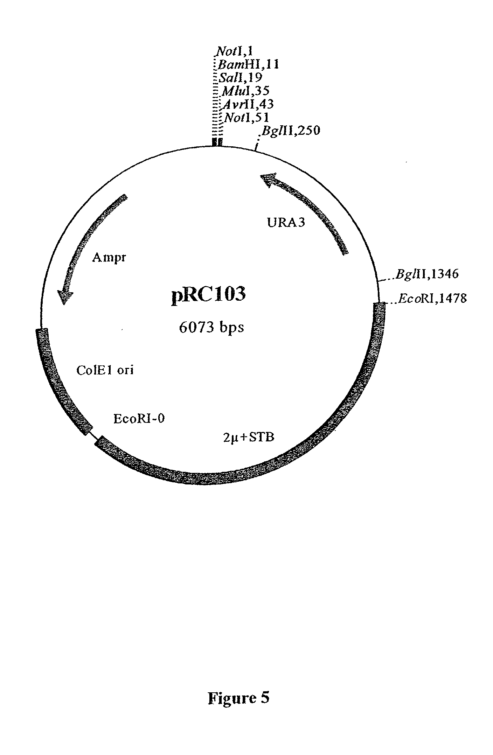

Le plasmide pRC103 a été construit après digestion partielle du pRC102 par l'endonucléase *EcoRI*, puis traitement à la Large Fragment of DNA Polymerase I (Fragment de Klenow, Life Technologies) et religation. Après avoir vérifié que le site *EcoRI* ainsi éliminé était bien celui adjacent à la séquence ColEl (origine de réplication d'*E.coli*), nous avons dénommé ce nouveau plasmide pRC103 (Figure 5).

Le plasmide pRC104 correspond à l'insertion du terminateur ter*PGK1* dans pRC103.

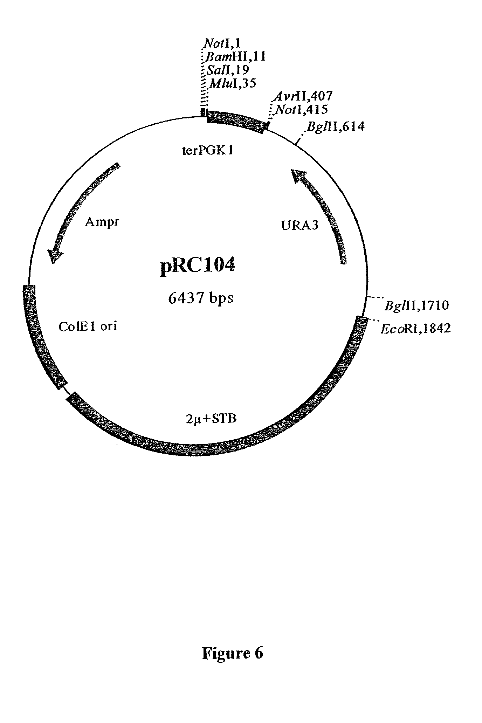

La PCR faite avec les oligonucléotides ORC107 et ORC108 a permis d'amplifier la séquence du terminateur ter*PGK1*. Toutes les amplifications ont été réalisées en utilisant la DNA polymérase AdvanTaq™ (Clontech) dans des conditions connues de l'homme de l'art. Le fragment obtenu, après purification, a été digéré par *MluI* et *AvrII.* Le clonage de cet insert dans le plasmide pRC103, préalablement digéré par ces mêmes enzymes de restriction, a permis d'obtenir pRC104 (Figure 6).

Le plasmide pRC105 correspond à l'insertion du promoteur pro*TEF1* dans pRC104.

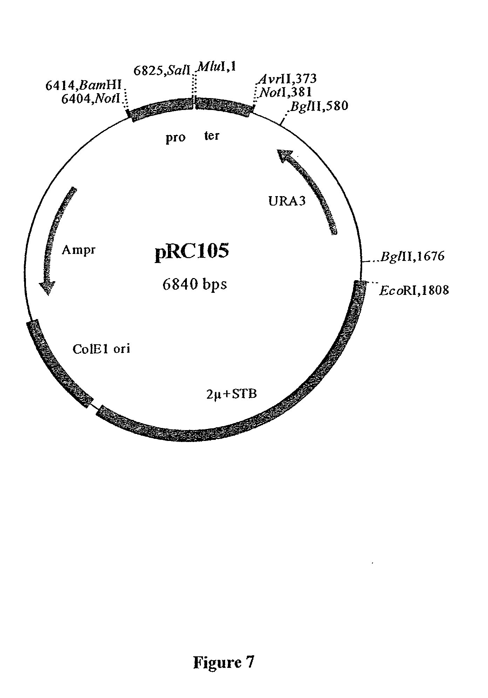

La PCR faite avec les oligonucléotides ORC103 et ORC104 a permis d'amplifier la séquence du promoteur pro*TEF1.* Le fragment obtenu, après purification, a été digéré par *BamHI* et *SalI*. Le clonage de cet insert dans le plasmide pRC104, préalablement digéré par ces mêmes enzymes de restriction, a permis d'obtenir pRC105 (Figure 7).

La PCR d'*ATF1* a été effectuée avec les oligonucléotides ORC129 et ORC130 sur un extrait d'ADN total isolé de la souche DCL "M" (Walker, 1998 ; achetée auprès de DCL, GB ; www.dclyeast.co.uk) selon le programme suivant : 5 minutes de dénaturation à 94°C, puis 27 cycles composés de 45 secondes de dénaturation à 94°C suivies d'1 minute d'hybridation à 55°C suivie d'1,5 minute d'élongation à 72°C, puis, à l'issue du 27^{ème} cycle, une élongation finale de 10 minutes à 72°C.

La PCR d'*ATF2* a été effectuée avec les oligonucléotides ORC139 et ORC140 sur un extrait d'ADN total isolé de la souche X2180-1A (obtenue auprès de la NCYC) selon le programme suivant : 5 minutes de dénaturation à 94°C, puis 20 cycles composés d'1 minute de dénaturation à 94°C suivie d'1 minute d'hybridation à 54,5°C suivie d'1,6 minute d'élongation à 70°C, puis, à l'issue du 20^{ème} cycle, une élongation finale de 10 minutes à 70°C.

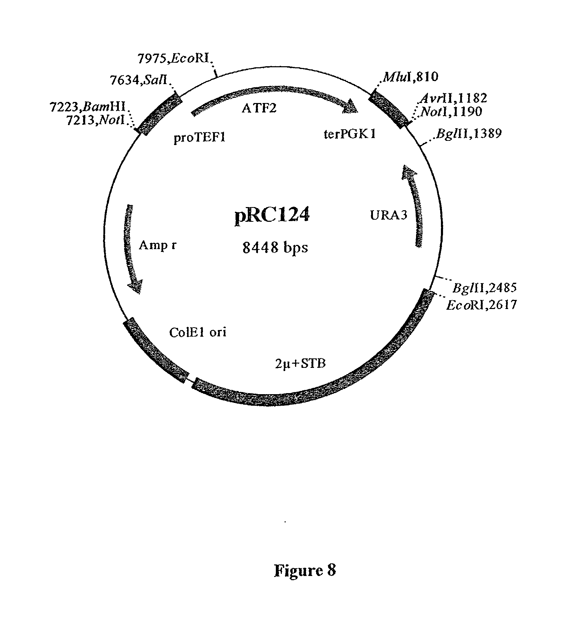

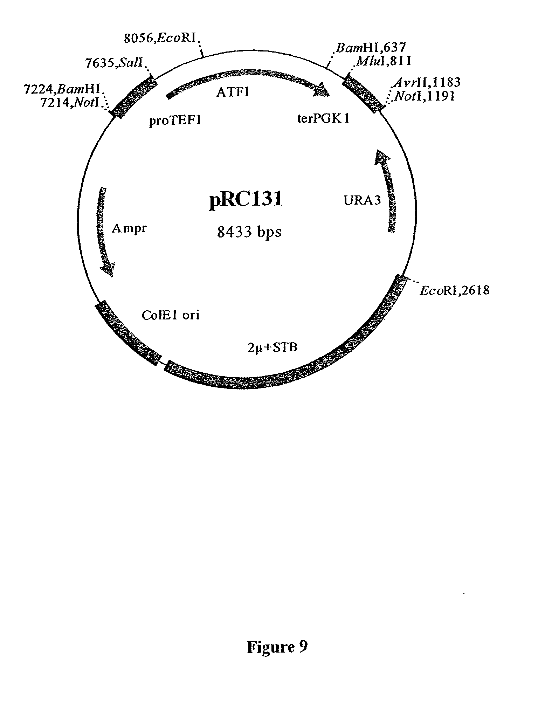

Les fragments de PCR, après purification par le Kit Concert™ Rapid PCR (Life Technologies), ont été mis à digérer par les endonucléases *SalI* et *MluI.* Les inserts ainsi obtenus ont pu être clonés dans le plasmide pRC105 préalablement soumis aux mêmes digestions par *SalI* et *MluI.* Le clonage dans pRC105 du fragment correspondant à la PCR d'*ATF2* a donné le plasmide pRC124 (Figure 8). Celui correspondant à la PCR d'*ATF1* a donné le plasmide pRC131 (Figure 9).

La transformation de ces plasmides dans la souche YRC988 (*ATF1, atf2, iah1*) *a* été effectuée selon des protocoles établis (Ito *et al*., 1983 ; Gietz *et al*., 1992, 1995).

### Fermentation sur malt.

Les souches à étudier sont précultivées sous agitation à 150 rpm pendant la nuit à 29°C. Ces précultures sont réalisées en fioles Erlenmeyer de 500 ml dans 100 ml de milieu liquide SC100 (Sherman, 1991 ; contenant 10% w/v de glucose) dépourvu d'uracile, ce qui répond aux conditions de sélection pour la présence du plasmide. Les cultures sont ensemencées comme décrit précédemment dans du milieu malt. Ce dernier est supplémenté en leucine, lysine et histidine ainsi qu'en G418. L'expression du gène *ATF1* étant fortement réprimée par des acides gras non saturés (Fuji *et al*., 1997 ; Fujiwara *et al*., 1998) et afin d'annuler l'expression de la copie génomique d'*ATF1*, le milieu a été additionné de Tween 80 à 0.25% v/v. Après 54 heures de fermentation, on mesure le profil métabolique comme précédemment (exemple 1).

### Dosage de l'acétate d'éthyle

La mesure d'acétate d'éthyle se fait sur 10 ml du milieu centrifugé. Les échantillons ont été analysés par chromatographie en phase gazeuse sur un appareil Agilent 6890. L'analyse se fait par le protocole suivant : Colonne CP-WAX 57 CB (Varian) 50 m par 250 µm de diamètre interne (pour 0,2 %µm d' épaisseur du film) ; programme de température: 40°C pendant 5 minutes, puis 5°C/minute jusqu'à 210°C. Les températures du détecteur et de l'injecteur sont maintenues à 250°C. L'injection de 1 µl se fait en mode « Split »1/10 pendant 30 secondes ; le débit de l'hélium dans la colonne est constant à 1.5 ml/minute. Les produits sont détectés à l'aide d'un détecteur à ionisation de flamme. La quantité d'une substance donnée est calculée relative à l'aire du standard interne (4-méthyl-2-pentanol).

### Résultats

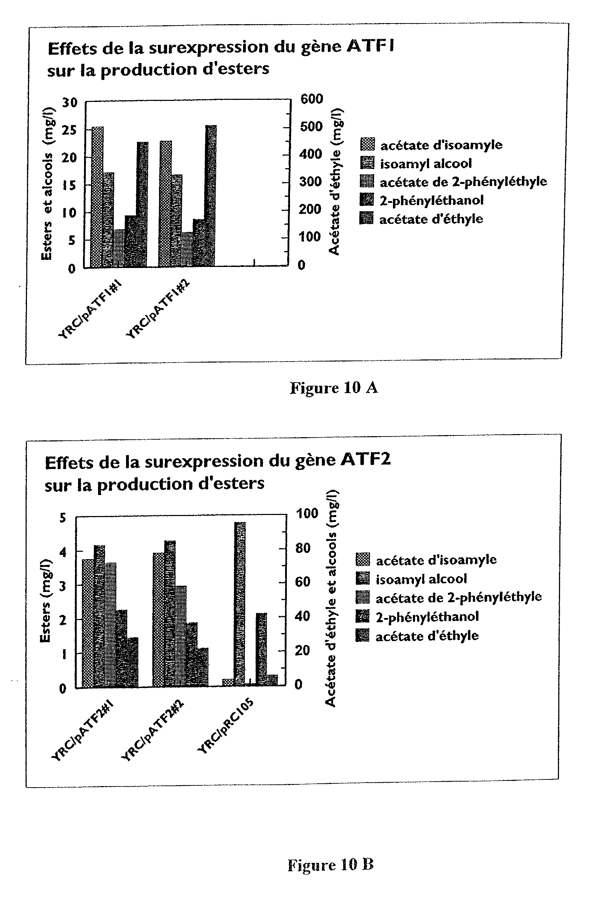

La souche YRC988 est transformée par des plasmides exprimant, sous contrôle du promoteur pro*TEF1,* soit le gène *ATF1* (pRC131), soit le gène *ATF2* (pRC124). Pour chaque gène, 2 clones sont choisis, qui seront appelés dans la Figure 10 YRC/pATF1#1 et #2 dans le cas d'*ATF1* et YRC/pATF2#1 et #2 dans le cas d'*ATF2.* Une souche servant de contrôle a été obtenue par transformation de YRC988 par un plasmide contenant le block d'expression pro*TEF1-*ter*PGK1* sans insert. Elle sera appelée YRC/pRC105 dans la Figure 10B.

Après fermentation durant 55 heures, les métabolites sont extraits comme précédemment décrit. Les résultats sont résumés dans la Figure 10A (surexpression d'*ATF1*) et Figure 10B (surexpression d'*ATF2* et souche contrôle).

Dans la Figure 10A on remarquera l'augmentation considérable, obtenue par la surexpression plasmidique d'*ATF1,* de la teneur en esters acétylés d'éthanol, d'isoamylalcool et de 2-phényléthanol. L'acétate d'éthyle atteint des niveaux jusqu'à 70 fois supérieurs à ceux de la souche contrôle (voir Figure 10B).

La surexpression d'*ATF2* produit au cours de la fermentation une accumulation d'acétate d'éthyle limitée à un facteur multiplicatif de 2 à 4, comparé au contrôle. Dans le cas de la surexpression d'*ATF2*, si les accumulations d'esters acétylés sont inférieures à celles d'une surexpression d'*ATF1,* on remarque toutefois des taux d'acétate multipliés par 50 à 60 pour le 2-phényléthyle et par 18 à 20 pour l'acétate d'isoamyle, comparativement au contrôle.

Dans le Tableau 3, sont repris les rapports IAAc/FEAc et acétate d' éthyle/FEAc obtenus pour les différentes souches. Comparé à la souche contrôle, le rapport IAAc/FEAc augmente lorsque la souche surexprime *ATF1* alors que ce rapport diminue lorsque la souche surexprime *ATF2*. Le rapport acétate d'éthyle/FEAc est 10 fois supérieur pour une souche qui surexprime *ATF1* comparé à celle qui surexprime *ATF2.* Il en résulte qu'en surexprimant *ATF2* plutôt qu'*ATF1* on obtient un produit plus équilibré d'un point de vue sensoriel, avec moins de substances indésirables (tel l'acétate d'éthyle, par exemple).

**Tableau 3**

| Souche (YRC988) transformée par : | Rapport : | |
|---|---|---|
| | IAAc/FEAc | Acétate d' éthyle/FEAc |
| PRC131 (*ATF1*) #1 | 3.73 | 67 |
| PRC131 (*ATF1*) #2 | 3.72 | 83 |
| | | |
| PRC124 (*ATF2*) #1 | 1.03 | 7.9 |
| PRC124 (*ATF2*) #2 | 1.33 | 7.6 |
| | | |
| PRC105 (-) #1 | 3.33 | 108 |

### Exemple 4: étude in vivo dans un milieu additionné d'alcools de l'activité alcool acétyle transférase à partir du gène ATF2 surexprimé.

### Outil

Transformation dans la souche YRC1001 (*atf1, atf2, iah1*) par le plasmide exprimant, sous contrôle du promoteur pro*TEF1*, le gène *ATF2* (pRC124).

### Fermentation sur malt

Les précultures sont réalisées dans du milieu liquide SC20 (Sherman, 1991) dépourvu d'uracile et selon la même procédure que dans l'exemple 3. Les cultures sont ensemencées comme décrit précédemment dans du milieu malt, cette fois non glucosé. Ce dernier est supplémenté en leucine, lysine et histidine. A ce milieu malt, on ajoute juste avant ensemencement des alcools à raison de 100 µg/ml. Après fermentation pendant 98 heures, on analyse -comme décrit précédemment- les alcools et leurs produits dérivés (esters acétylés de l'alcool, l'acide correspondant à l'alcool et son ester éthylé). La capacité de bioconversion est ensuite exprimée par le rapport « ester acétylé de l'alcool ajouté »/« somme des alcools et produits dérivés ». Cette valeur donne une indication de la capacité d'acétyler les différents alcools ajoutés au milieu extérieur par la souche de levure transformée. Les résultats sont repris dans le Tableau 4.

On constate qu'une souche surexprimant *ATF2* montre une activité accrue avec des alcools à chaîne longue, avec des substrats qui ont un noyau hydrophobe ou encore avec des substrats terpéniques. Mais elle ne semble pas capable d'acétyler un alcool tertiaire (linalool). Notons en particulier l'apport aromatique des esters acétylés de terpènes, tel le β-citronellol, le géraniol.

**Tableau 4**

| Alcool ajouté | Bioconversion par YRC1001/pRC124 |
|---|---|
| n-butanol | 0,021 |
| isoamylalcool | 0,045 |
| n-hexanol | 0,046 |
| n-heptanol | 0,074 |
| n-octanol | 0,078 |
| n-décanol | 0,086 |
| | |
| 2-phényléthanol | 0,128 |
| benzylalcool | 0,163 |
| géraniol | 0,28 |
| β-citronellol | 0,71 |
| linalool | 0 |

### Exemple 5 : étude in vivo de l'ajout de phénylalanine sur l'activité alcool acétyle transférase à partir du gène ATF2 surexprimé.

Transformation dans la souche YRC1001 (*atf1, atf2, iah1*) par le plasmide exprimant, sous contrôle du promoteur pro*TEF1,* le gène *ATF2* (pRC124).

### Fermentation sur jus de raisin blanc

Des précultures sont ensemencées pour la nuit à 29° C par les souches à étudier, dans le milieu YNBD (Sherman, 1991) additionné des exigences des souches en acides aminés. La sélection pour la présence du plasmide est effectuée par l'absence d'uracil dans le milieu de préculture.

Les cultures sont ensemencées à une densité optique mesurée à 578 nm de 0.1 (équivalent à 10⁶ cellules/ml) en fioles Erlenmeyer de 150 ml contenant 150 ml de jus de raisin blanc. Le milieu jus de raisin blanc est obtenu à partir d'un jus de raisin blanc concentré (CSR Pampryl) à raison d'1 volume pour 3,4 volumes d'eau déminéralisée. Le milieu est stérilisé pendant la nuit par ajout de 150 µl de diéthyle pyrocarbonate (DEPC, Sigma). La fermentation se fait pendant 312 heures (13 jours) à 29° C. Après fermentation en jus de raisin blanc, les profils métaboliques ont été analysés comme précédemment. L'influence de l'addition de phénylalanine (à raison de 20 ou de 100 mg/l) sur la production d'acétate de phényle éthyle est mesurée.

### Résultats

Les données sont reprises dans le tableau 5. On s'aperçoit qu'un milieu plus riche en phénylalanine (précurseur métabolique de phényl éthanol) est favorable à une production accrue d'acétate de phényle éthyle alors que le niveau d'acétate d'isoamyle reste faible. Par contre, lorsque la souche surexprime le gène *ATF2* à partir d'un plasmide, non seulement il y a formation d'esters acétylés, parmi lesquels l'augmentation de la production d'acétate de phényle éthyle est remarquable.

En particulier, le taux d'acétate de 2-phényléthyle est multiplié par un facteur 80 lorsque l'on compare une souche qui surexprime le gène *ATF2* à une souche contrôle dans un milieu additionné de 100 mg/ml de phénylalanine.

**Tableau 5**

| Souche | YRC1001/pRC105 | | | YRC1001/pRC124 | | |
|---|---|---|---|---|---|---|
| Addition au milieu : | | | | | | |
| Phénylalanine* | - | 20 | 100 | - | 20 | 100 |
| | | | | | | |
| Isoamyle alcool | 33 | 30.3 | 34 | 25.5 | 27.6 | 23.9 |
| Acétate d'isoamyle | 0 | 0 | 0 | 0.2 | 0.2 | 0.7 |
| | | | | | | |
| Phényle alcool | 17.5 | 21.9 | 64.7 | 12.3 | 23.7 | 49.4 |
| Acétate de phényle alcool | 0 | 0.02 | 0.1 | 1.1 | 2.8 | 8.3 |

| | | | | | | |
|---|---|---|---|---|---|---|
| * en mg/l | | | | | | |

### Exemple 6

Fermentation en jus de raisin blanc (CSR Pampryl ; 100% pur jus sans concentré Raisin blanc et muscat) avec mesure des métabolites produits et appréciation sensorielle. Etude de la surexpression du gène *ATF2* ou *ATF1* dans une souche produisant naturellement des terpènes (100x145), décrite notamment dans le document FR 2 622 208, en particulier dans les exemples 1 à 6.

### Construction d'un plasmide kanR-ATF2, pRC172.

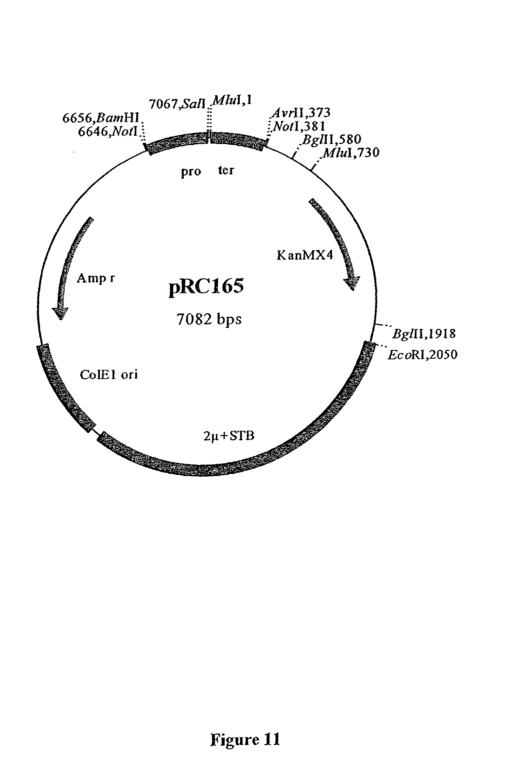

Une PCR réalisée avec les oligonucléotides ORC169 et ORC170 [ ORC 169 (*kanR* FW) 5'AAAAAGATCTGATGTGACTGTCGCCCGTACATT3' et ORC 170 (*kanR* RV) 5'AAAAAGATCTCACTGGATGGCGGCGTTAGTAT3'] sur le plasmide pFA6a (Wach *et al*., 1994) a permis d'obtenir un fragment de 1352 pb correspondant au gène *kanR*, bordé par deux sites *BglII.* Ce fragment, après digestion par *BglII*, a été inséré dans le plasmide pRC105 préalablement libéré du marqueur *URA3* par une digestion par *BglII*. Ceci génère le plasmide pRC165, dans lequel le marqueur *URA3* a été remplacé par *kanR* (Figure 11). Le sens d'insertion a été vérifié par *ClaI*. Le plasmide pRC172 a été construit par remplacement, dans pRC165, de la cassette pro*TEF1-*ter*PGK1* par la cassette pro*TEF1-ATF2-*ter*PGK1* (cassette d'expression d'*ATF2* sous contrôle du promoteur pro*TEF1* et du terminateur ter*PGK1*). Cette dernière a été isolée par *NotI* du plasmide pRC124 (décrit plus haut), puis insérée dans pRC165 préalablement libéré par *NotI* de sa cassette pro*TEF1-*ter*PGK1.* Le sens d'insertion a été déterminé par *EcoRI.*

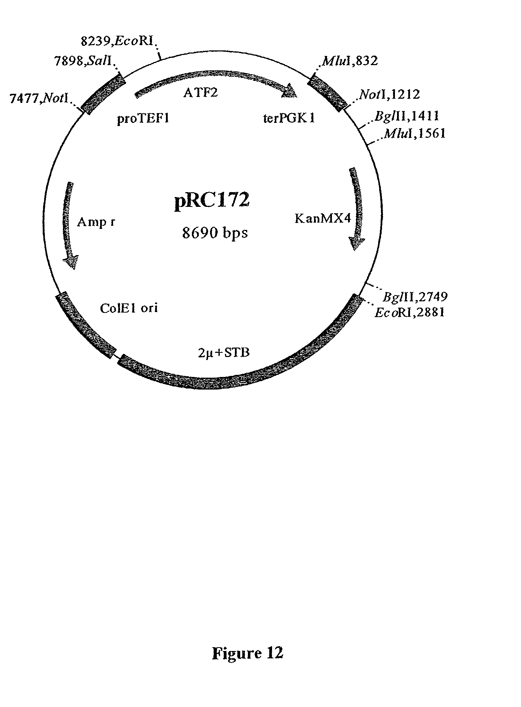

Le plasmide pRC172 (Figure 12) contient la cassette d'expression du gène *ATF2* sous contrôle du promoteur pro*TEF1* et du terminateur ter*PGK1.* Le plasmide porte le gène marqueur *kanR*, conférant à la levure la résistance à la généticine (G418).

### Construction d'un plasmide kanR-ATF1, pRC169.

Le plasmide pRC169 a été construit par remplacement, dans pRC165, de la cassette pro*TEF1-*ter*PGK1* par la cassette pro*TEF1-ATF1*-ter*RGK1* (cassette d'expression d'*ATF1* sous contrôle du promoteur pro*TEF1* et du terminateur ter*PGK1*). Cette dernière a été isolée par *NotI* du plasmide pRC131 (décrit plus haut), puis insérée dans pRC165 préalablement libéré par *NotI* de sa cassette pro*TEF1-*ter*PGK1.*

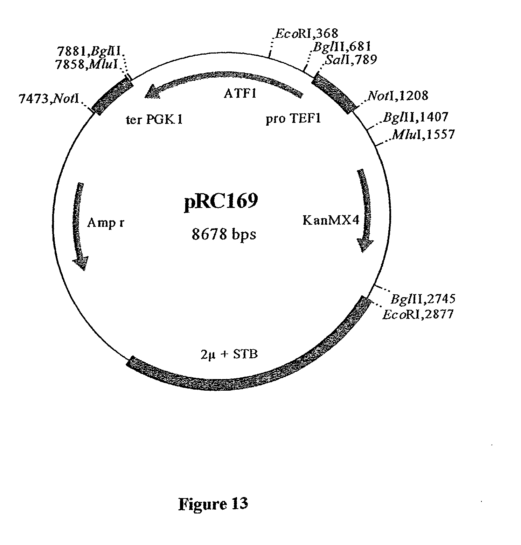

Le plasmide pRC169 (Figure 13) contient la cassette d'expression du gène *ATF1* sous contrôle du promoteur pro*TEF1* et du terminateur ter*PGK1.* Le plasmide porte le gène marqueur *kanR*, conférant à la levure la résistance à la généticine (G418). Les orientations relatives sont identiques à celles du plasmide pRC172, à l'exception de la cassette d'expression *ATF1.*

La transformation des plasmides (pRC165, pRC172 et pRC169) dans la souche 100x145 (Javelot *et al*., 1991) a été effectuée selon des protocoles établis (Ito *et al.,* 1983 ; Gietz *et al*., 1992, 1995). La souche 100x145 transformée par le plasmide pRC169 a été déposée sous le numéro CNCM I-2741, La souche 100x145 transformée par le plasmide pRC169 a été déposée sous le numéro CNCM I-2741, la souche 100x145 transformée par le plasmide pRC172 a été déposée sous le numéro CNCM I-2742.

La souche non-transformée servira de contrôle. Pour chaque plasmide, 2 clones sont analysés, donnant des résultats semblables.

### Fermentation sur jus de raisin blanc.

Des précultures sont ensemencées pour la nuit à 25°C par les souches à étudier, dans le milieu YPD100 (Sherman, 1991 ; contenant 10% w/v de glucose), additionné de Tween 80 (0.05% v/v) et ergostérol (10 mg/l). Dans le cas des souches transformées et afin d'exercer une sélection pour la présence du plasmide on ajoute au milieu de préculture 200 µg/ml de G418. Les cellules sont centrifugées et reprises dans un volume d'eau stérile approprié afin de servir d'inoculum. Les cultures sont ensemencées à une densité optique mesurée à 578 nm de 0.5 (équivalent à 0.5 10⁷ cellules/ml) en fioles Erlenmeyer de 150 ml contenant 150 ml de jus de raisin blanc. Le « jus de raisin blanc » (CSR Pampryl) est obtenu dans un super marché local en bouteilles de 1 litre (jus de raisin blanc contenant 12% v/v de muscat d'Alexandrie). La fermentation se fait pendant 15 jours à 25°C. Après fermentation en jus de raisin blanc, les profils métaboliques ont été analysés comme précédemment.

### Dosage des sucres

Les principaux métabolites produits (éthanol, glycérol) ainsi que les sucres résiduels (glucose, fructose) ont été mesurés par HPLC (Colonne Aminex HPX-87H 30 cm x 4,5 cm (Bio-Rad) maintenue à 65 °C. Détecteur refractométrique. Phase mobile : H₂SO₄ 0,005M, débit : 0,7ml/mn). Les produits à analyser ont été détectés par leur temps de rétention en comparaison avec celui de la substance pure. Les concentrations ont été calculées après injection des composés en quantités connues.

### Dosage des métabolites : Acide acétique : acétaldéhyde.

Ces métabolites sont dosés par kit enzymatique Roche (Numéro du catalogue : 148261 et 668613, respectivement) selon les instructions du fournisseur.

### Dosage des métabolites volatils :

Les alcools supérieurs, les terpènes et leurs dérivés acétylés sont déterminés par chromatographie en phase gazeuse comme décrit ci-haut.

### Dégustation

L'évaluation sensorielle a été effectuée par des professionnels.

### Résultats

Les données biochimiques sont reprises dans le Tableau SA. On s'aperçoit que comparées aux souches contrôles, les souches transformées qui expriment le gène *ATF1* ou *ATF2* produisent plus de dérivés acétylés (en particulier des esters d'isoamylalcool, de 2-phényléthanol et de géraniol) dans le jus fermenté. En particulier, il semblerait que la présence simultanée d'acétate de géranyle et de géraniol (entre autres) soit corrélée avec la perception muscat d'une complexité plus grande de la boisson fermentée (Tableau 5B). A noter que l'accumulation d'acétate d'éthyle est moins importante (au moins 4 fois) dans les souches exprimant *ATF2* que celles exprimant *ATF1.* Aussi, vis à vis des souches contrôles, l'augmentation de la concentration en acétate d'éthyle chez les souches qui expriment *ATF2*, n'est que d'un facteur 2. En test sensoriel (Tableau 5B), les jus de raisin fermentés par la souche exprimant *ATF1* sont perçus comme agressifs, ce qui est dû à une note de solvant, d'éthyle acétate. Il en résulte qu'en surexprimant *ATF2* on obtient un produit plus équilibré, fruité grâce aux esters acétylés, avec moins de substances indésirables d'un point de vue sensoriel.

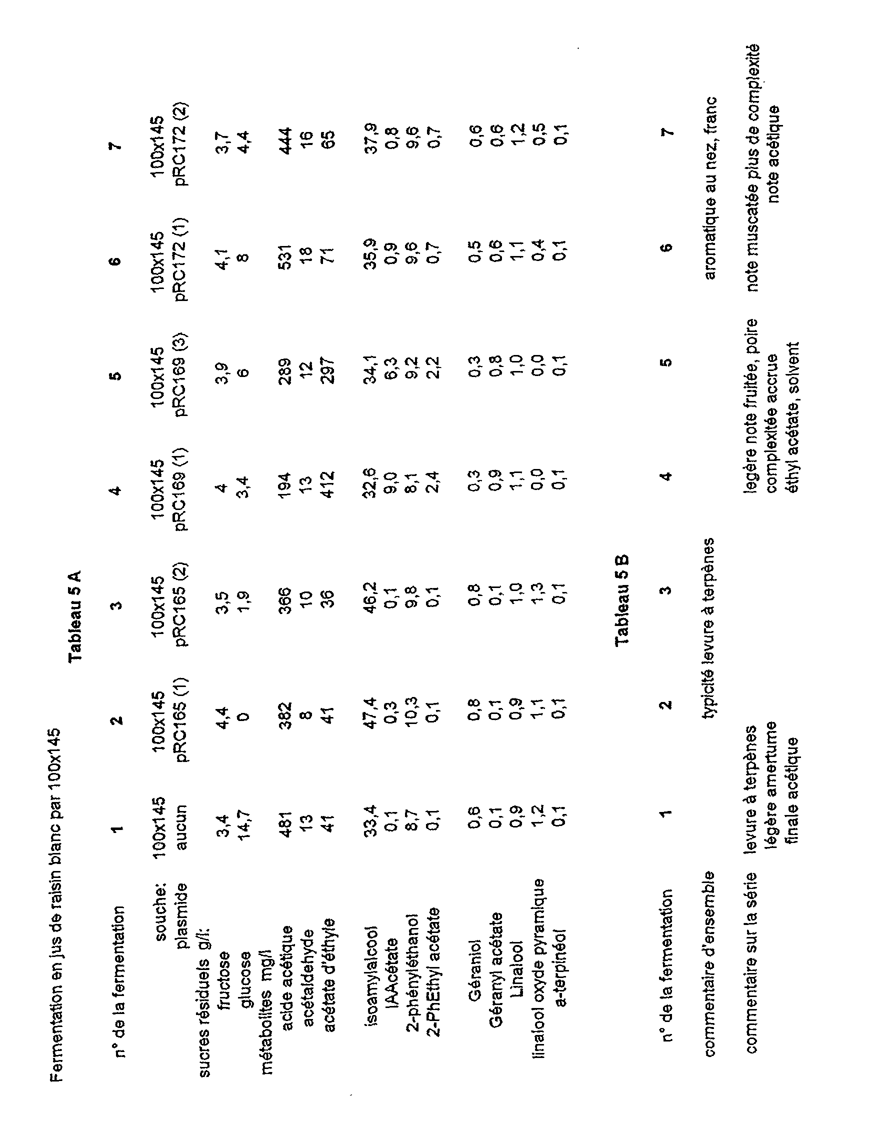

### Exemple 7

Fermentation en jus de raisin blanc (CSR Pampryl ; 100% pur jus sans concentré Raisin blanc et muscat) avec mesure des métabolites produits et appréciation sensorielle. Etude de la surexpression du gène *ATF2* ou *ATF1* dans une souche oenologique .

### Construction de la souche YRC1342

La souche L116 est une souche oenologique (Javelot C., Girard P., Colonna-Ceccaldi B. et Vladescu B. 1991 J. Biotechnol. 21 p 239-252) pour laquelle des spores ont été isolées et décrites. Les spores 5b et 7a présentent un signe sexuel stable et, croisées entre elles, redonnent des caractéristiques fermentaires comparables à celles de la souche parentale L116.

Construction d'un plasmide contenant un gène *URA3* délété d'une partie interne *(URA3Δ)* 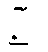

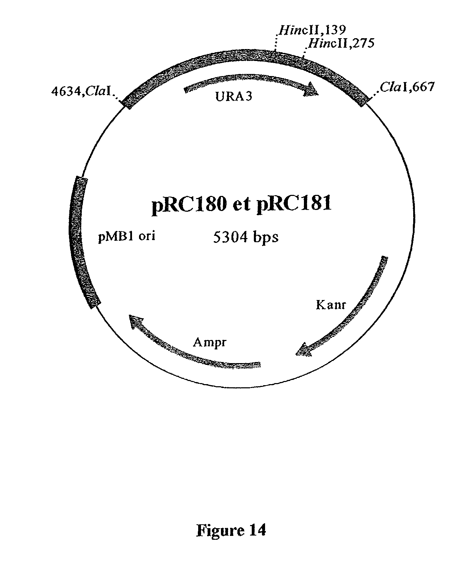

L'ADN des spores de 5b et 7a de la souche L116 a été extrait. Les oligonucléotides (ORC59: 5' AAATCGATGCGAGGCATATTTATGGTGAAGGATAAG 3' et ORC60: 5' AAATCGATGTCTTATTGTTCTTGATTTGTGCCCCGTA 3') utilisés pour l'amplification du gène *URA3* portent un site de restriction *Cla*I à leur extrémité 5'. Les PCR effectuées sur l'ADN génomique de 5b, d'une part, et de 7a, d'autre part, ont donc permis d'obtenir des produits contenant le gène *URA3* bordé par des sites de restriction *Cla*I. Après ligation dans le vecteur bactérien pCR4®Blunt-TOPO® (Invitrogen) et transformation bactérienne, 2 clones, l'un correspondant au clonage d'*URA3* de 5b (pRC180), l'autre correspondant au clonage d'*URA3* de 7a (pRC181) ont été construits (Figure 14).

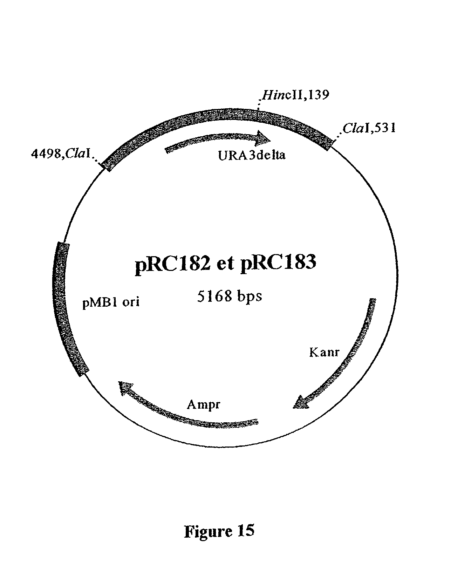

La coupure par *Hinc*II des plasmides pRC180 et 181 a permis de libérer un fragment de 136 pb, interne à l'ORF d'*URA3.* Après ligature des vecteurs digérés par *Hin*cII et transformation bactérienne, 2 nouveaux plasmides (pRC182 et pRC183, Figure 15) ont été isolés, issus respectivement des plasmides pRC180 et pRC181.

Une digestion par *Cla*I a permis d'isoler le fragment *URA3*Δ sur gel.

### Construction d'un plasmide centromérique avec un marqueur de sélection positif.

Le gène de résistance à la kanamycine (*kanR*) a été amplifié à partir du plasmide pFA6a-KANMX4 (décrit par Wach *et al*., 1994. Les oligonucléotides utilisés sont le ORC61 et ORC62.

ORC61 : 5' AAAGATATCGATGTGACTGTCGCCCGTACA 3' et
ORC62 : 5' AAAGATATCGTATTACCGCCTTTGAGTGAGCTGA 3'

Le produit de PCR du gène *kanR* a été cloné dans le plasmide pCR4®Blunt-TOPO®.

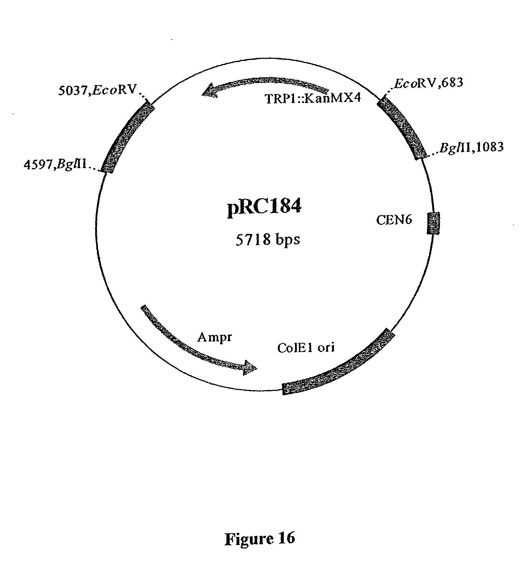

Les transformants bactériens ont été directement sélectionnés sur kanamycine (50 µg/ml). Le fragment *Eco*RV*-Eco*RV contenant *kanR* a ensuite été introduit au site *Eco*RV du gène *TAP1* porté par le plasmide de levure pFL39 (Bonneaud et al., 1991). 4 clones de ce nouveau plasmide pRC184 ont été isolés et contrôlés (Figure 16). Ce nouveau vecteur monocopie (centromérique) porte le gène de résistance au G418 (*kanR*) inséré dans la séquence codant pour *TAP1.*

### Construction de souches URA3Δisogéniques de L116

Les spores 5b et 7a compétentes (voir ci-haut) sont co-transformées par 1.25 µg du fragment *URA3*Δ isolé respectivement de pRC 182 et de pRC183 ainsi que 2 µg du plasmide pRC184. Après sélection sur milieu YPD + G418 (200µg/ml), les colonies obtenues sont repiquées sur un milieu SD sélectif, préparé selon Boeke et coll., contenant l'acide 5-fluoro-orotique (5FOA). Les clones qui sont auxotrophes pour l'uracile et résistants au SFOA sont propagés sur milieu YPD et dilués sur ce même milieu gélosé afin d'obtenir des colonies isolées. Celles-ci sont repiquées sur YPD et YPD + G418 (200 µg/ml). Les clones qui sont devenus sensibles au G418 sont retenus : YRC1008 (*URA3Δ,* isogénique de 5b) et YRC1009 et YRC1013 (*URA3Δ* isogénique de 7a). Après croisement de YRC1008 par YRC1009 ou par YRC1013 on obtient les souches YRC1342 et YRC1344 respectivement qui sont *URA3Δ*et isogéniques de la souche 5bx7a, issue de L116.

Les plasmides pRC105, pRC124 et pRC131 (décrits plus-haut) sont transformés dans la souche YRC1342 en sélectionnant une prototrophie pour l'uracile sur milieu YNBD. Les souches YRC1342 transformées par le plasmide pRC124 et le plasmide pRC131 ont été déposées respectivement sous les numéros CNCM I-2739 et 1-2740.

### Fermentation sur jus de raisin blanc.

Des précultures sont ensemencées pour la nuit à 25°C par les souches à étudier, dans le milieu YPD100 (Sherman, 1991 ; contenant 10% w/v de glucose). Les cellules sont centrifugées et reprises dans un volume d'eau stérile approprié afin de servir d'inoculum. Le « jus de raisin blanc » (CSR Pampryl) est obtenu dans un super marché local en bouteilles de 1 litre (jus de raisin blanc contenant 12% v/v de muscat d'Alexandrie). Une solution d'enzymes pectolytiques est préparée (AR 2000 distribué par Gist-Brocades, DSM SA) à raison de 100 mg/ml et filtré sur Millipore 0.45 µm. Cette préparation est ajoutée à 0.1% v/v dans le jus de raisin. Les cultures sont ensemencées à une densité optique mesurée à 578 nm de 0.5 (équivalent à 0.5 10⁷cellules/ml) en fioles Erlenmeyer de 150 ml contenant 150 ml de jus de raisin blanc. La fermentation se fait pendant 10 jours à 25°C. Après fermentation en jus de raisin blanc, les profils métaboliques ont été analysés comme précédemment.

### Dégustation

L'évaluation sensorielle a été effectuée en conditions standard par des professionnels.

### Résultats

Les données biochimiques sont reprises dans le Tableau 6A. On s'aperçoit que comparées aux souches contrôles, les souches transformées qui expriment le gène *ATF1* ou *ATF2* produisent plus de dérivés acétylés (en particulier des esters d'isoamylalcool, de 2-phényléthanol mais pas du géraniol) dans le jus fermenté. A noter que l'accumulation d'acétate d'éthyle est moins importante (au moins 3 fois) dans les souches exprimant *ATF2* que celles exprimant *ATF1.* En test sensoriel (Tableau 6B), les jus de raisin fermentés par la souche exprimant *ATF1* sont perçus comme agressifs, ce qui est dû à une note de solvant, d'éthyle acétate. Il en résulte qu'en surexprimant *ATF2* on obtient un produit plus équilibré, fruité grâce aux esters acétylés, avec moins de substances indésirables d'un point de vue sensoriel.

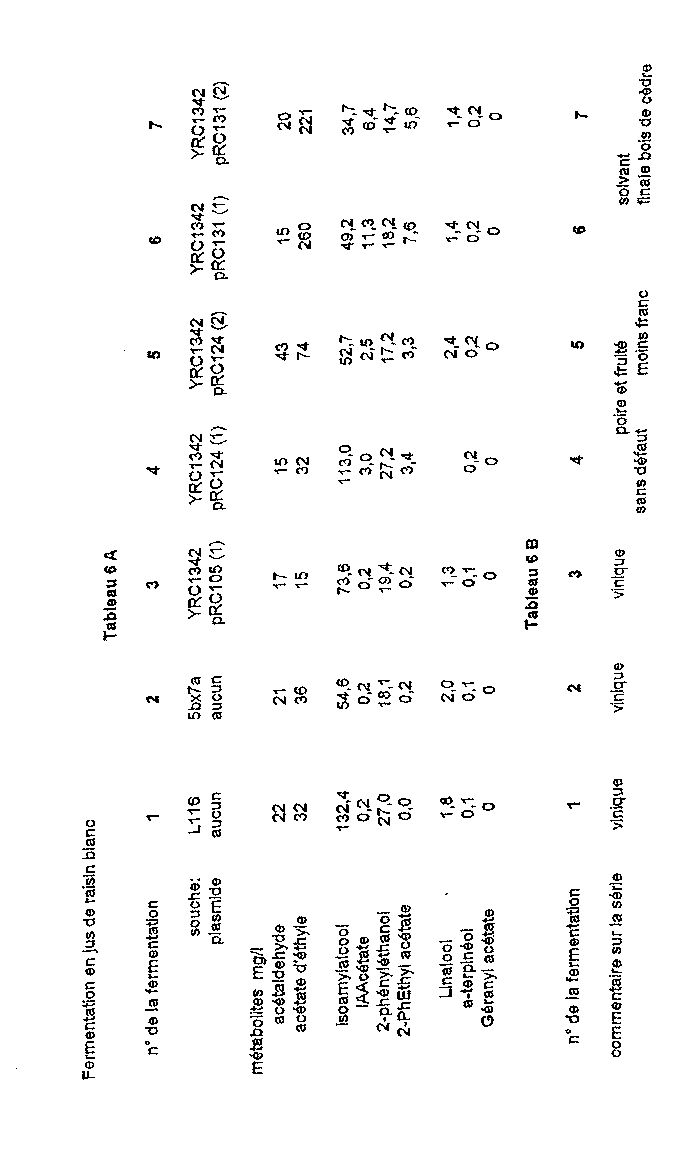

### Exemple 8

Fermentation en jus de raisin blanc (jus de raisin : Muscat d'Alexandrie ; Foulon Sopagly; CSR Pampryl) avec mesure des métabolites produits et appréciation sensorielle. Etude de la surexpression du gène *ATF2* ou *ATF1* dans une souche oenologique.

La souche YRC1342 est décrite ci-haut. Les plasmides pRC105, pRC124 et pRC131 (décrits plus-haut) sont transformés dans la souche YRC1342 en sélectionnant une prototrophie pour l'uracile sur milieu YNBD.

### Fermentation sur jus de raisin blanc.

Des précultures sont ensemencées pour la nuit à 25°C par les souches à étudier, dans le milieu YPD100 (Sherman, 1991 ; contenant 10% w/v de glucose). Les cellules sont centrifugées et reprises dans un volume d'eau stérile approprié afin de servir d'inoculum. Le jus de raisin blanc, Muscat d'Alexandrie est obtenu auprès de Foulon, Sopagly (CSR Pampryl). Une solution d'enzymes pectolytiques est préparée (AR 2000 distribué par Gist-Brocades) à raison de 100 mg/ml et filtrée sur Millipore 0.45 µm. Cette préparation est ajoutée à 0.1% v/v dans le jus de raisin. Les cultures sont ensemencées à une densité optique mesurée à 578 nm de 0.5 (équivalent à 0.5 10⁷ cellules/ml) en fioles Erlenmeyer de « 150 ml » contenant 150 ml de jus de raisin blanc. La fermentation se fait pendant 10 jours à 25°C. Après fermentation en jus de raisin blanc, les profils métaboliques ont été analysés comme précédemment.

### Dégustation

L'évaluation sensorielle a été effectuée en conditions standard par des professionnels.

### Résultats

Les données biochimiques sont reprises dans le Tableau 7A. On s'aperçoit que comparées aux souches contrôles, les souches transformées qui expriment le gène *ATF1* ou *ATF2* produisent plus de dérivés acétylés (en particulier des esters d'isoamylalcool, de 2-phényléthanol et du géraniol) dans le jus fermenté. A noter que l'accumulation d'acétate d'éthyle est moins importante (au moins 4 fois) dans les souches exprimant *ATF2* que celles exprimant *ATF1.* En test sensoriel (Tableau 7B), les jus de raisin fermentés par la souche exprimant *ATF1* sont perçus comme agressifs, ce qui est dû à une note de solvant, d'éthyle acétate. Cette note de solvant par sa dominance cache le fond muscaté de la boisson fermentée. Il en résulte qu'en surexprimant *ATF2* on obtient un produit plus équilibré, fruité grâce aux esters acétylés, avec moins de substances indésirables d'un point de vue sensoriel.

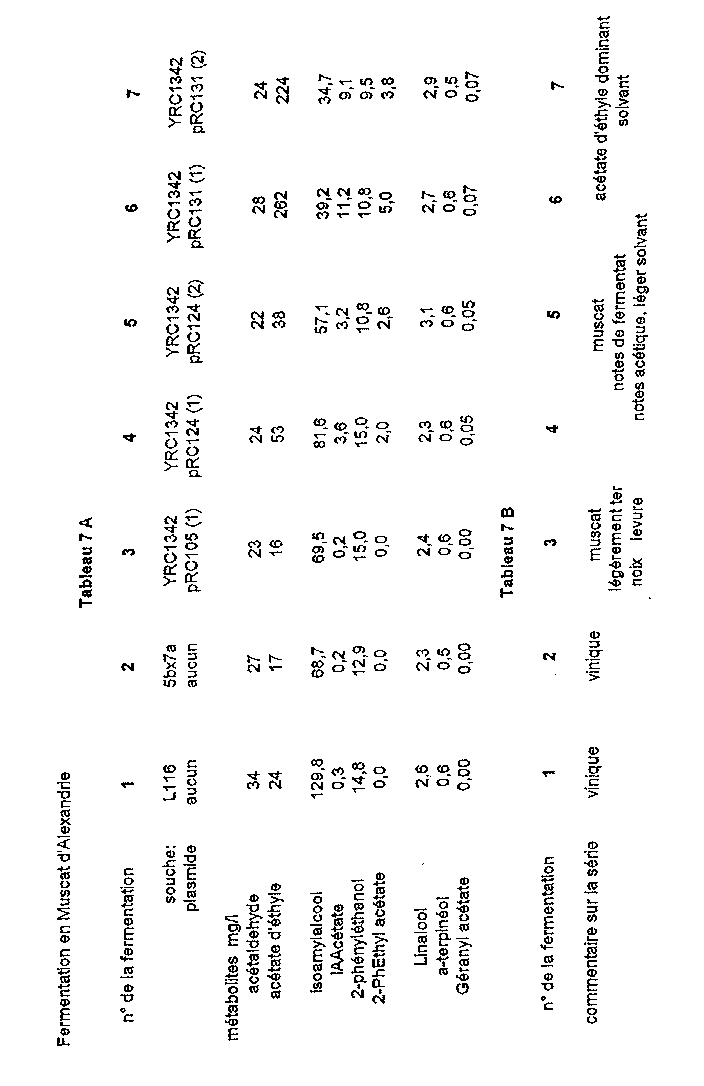

### Exemple 9

Fermentation en jus de raisin blanc avec mesure des métabolites produits et appréciation sensorielle. Etude de la surexpression du gène *ATF2* ou *ATF1* dans une souche oenologique.

### Fermentation sur jus de raisin blanc (Pampryl ; 100% pur jus sans concentré Raisin blanc et muscat).

Des précultures sont ensemencées pour la nuit à 25°C par les souches à étudier, dans le milieu YPD100 (Sherman, 1991 ; contenant 10% w/v de glucose). Les cellules sont centrifugées et reprises dans un volume d'eau stérile approprié afin de servir d'inoculum. Le milieu de préculture ou de culture destiné aux souches auxotrophes pour l'uracile est additionné de 60 mg/l d'uracile. Le « jus de raisin blanc» (CSR Pampryl) est obtenu dans un super marché local en bouteilles de 1 litre (jus de raisin blanc contenant 12% v/v de muscat d'Alexandrie). Une solution d'enzymes pectolytiques est préparée (AR 2000 distribué par Gist-Brocades) à raisin de 100 mg/ml et filtrée sur Millipore 0.45 µm. Cette préparation est ajoutée à 0.1% v/v dans le jus de raisin. Les cultures sont ensemencées à une densité optique mesurée à 578 nm de 0.5 (équivalent à 0.5 10⁷cellules/ml) en fioles Erlenmeyer de 3 L contenant 3 L de jus de raisin blanc. La fermentation se fait pendant 15 jours à 25°C. Après fermentation en jus de raisin blanc, les profils métaboliques ont été analysés comme précédemment. Les jus de raisin sont ensuite centrifugés et le surnageant est stocké à 4°C jusqu'à l'évaluation sensorielle.

### Dégustation

L'évaluation sensorielle a été effectuée en conditions standard par des professionnels.

### Résultats

Les données biochimiques sont reprises dans le Tableau 8. On s'aperçoit que comparées aux souches contrôles, les souches transformées qui expriment le gène *ATF1* ou *ATF2* produisent plus de dérivés acétylés (en particulier des esters d'isoamylalcool, de 2-phényléthanol) dans le jus fermenté.

Dans leur ensemble les résultats sont similaires à ceux obtenus à plus petite échelle dans l'exemple 7. Notons toutefois que les valeurs absolues des esters acétylés sont plus élevées dans l'exemple 9. Les écarts dans les valeurs absolues pour ces esters peuvent être dus à des différences dans les volumes fermentés et à la volatilité de ces composés rendant plus difficile une mesure absolue. A noter que l'accumulation d'acétate d'éthyle est moins importante (au moins 5 fois et se situe au même niveau que les souches contrôles) dans les souches exprimant *ATF2* que celles exprimant *ATF1.* En test sensoriel (Tableau 9), les jus de raisin fermentés par la souche exprimant *ATF1* sont perçus comme agressifs, ce qui est dû à une note de solvant, d'éthyle acétate. Cette note de solvant par sa dominance cache le fond muscaté de la boisson fermentée. Il en résulte qu'en surexprimant *ATF2* on obtient un produit plus équilibré, fruité grâce aux esters acétylés, avec moins de substances indésirables d'un point de vue sensoriel.

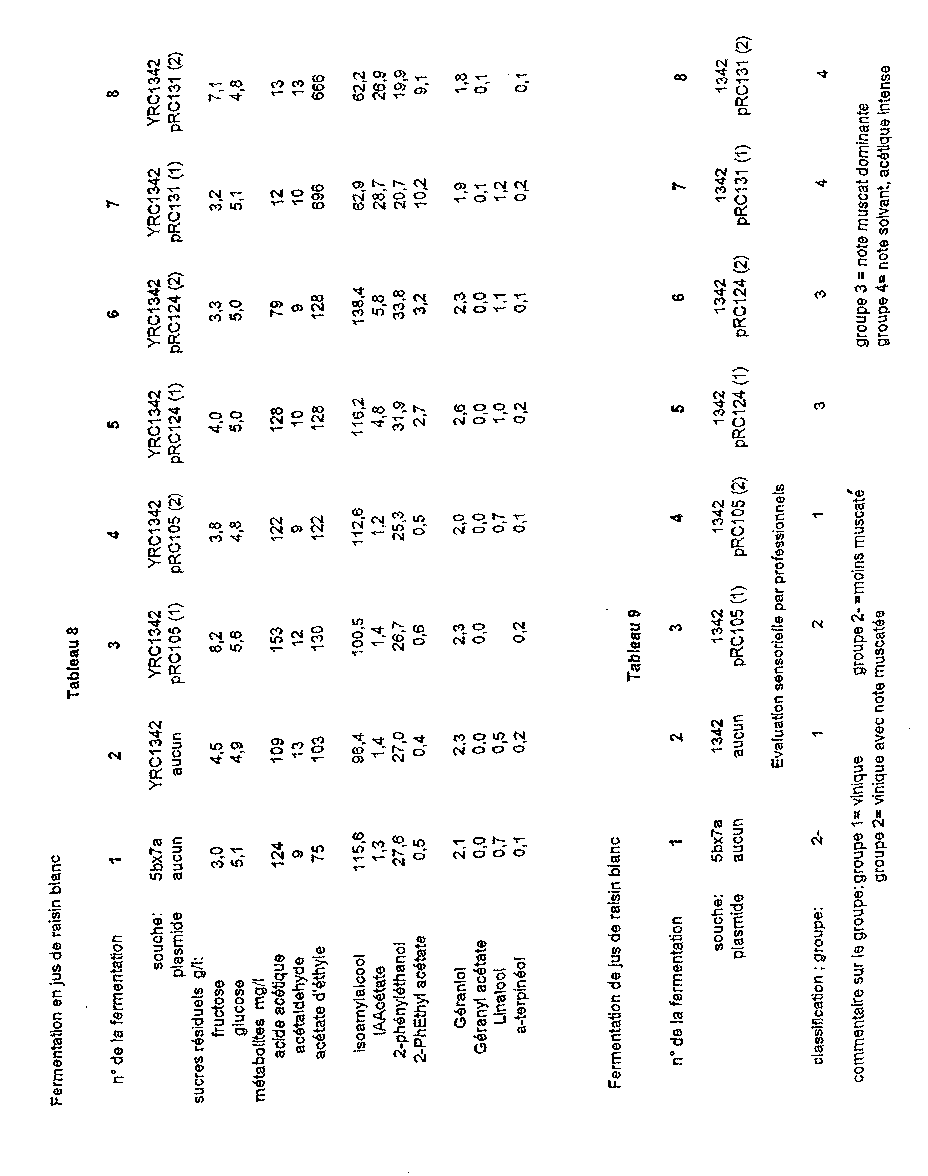

Grâce aux travaux illustrés par les neuf exemples décrits, les inventeurs ont amélioré la connaissance des mécanismes de production d'esters acétylés, en particulier en montrant :
- que *ATF2* est largement responsable de la production des esters acétylés dans une souche mutante *atf1*
- que le spectre d'activité d'Atf2p est différent de celui d'Atf1p ; en particulier Atf2p est moins active que Atf1p sur l'éthanol, l'isoamylalcool que sur le 2-phényléthanol ou les terpènes
- qu'une souche exprimant *ATF2* est capable d'acétyler un grand nombre d'alcools, de terpènes et de diols
- que des souches vinicoles sont capables de surexprimer *ATF2* et qu'elles produisent plus d'esters acétylés.

En outre les inventeurs sont allés en outre à l'encontre d'un préjugé technique en obtenant des boissons dont la valeur aromatique dépend surtout de l'équilibre entre ses composantes. En effet, en matière de procédé, on pourrait considérer que *ATF2 a* l'inconvénient de coder pour une protéine moins active que l'enzyme codée par *ATF1* : comparé à une souche sauvage, les rapports des composantes aromatiques acétylées évoluent dans un sens opposé en surexprimant *ATF2* au lieu de *ATF1*. Les inventeurs ont ainsi surexprimé le gène *ATF2* qui code pour une enzyme dont le spectre d'activité est différent de l'enzyme codée par *ATF1.* D'ailleurs, grâce à ce meilleur équilibre des composantes aromatiques, il n'est pas nécessaire d'utiliser une boisson supplémentée ou riche en précurseurs aromatiques.

Le procédé a de plus l'avantage pour une souche de levures donnée, d'orienter de manière souhaitée le métabolisme propre de la levure, en produisant des esters acétylés souhaités tels que les ceux dérivés du 2-phényléthanol, qui apportent des notes fruitées, fleuries, rosées, miel.

Le procédé peut également utiliser des souches mutantes qui surproduisent des précurseurs métaboliques des esters acétylés souhaités, tels que le 2-phényléthanol, de telles souches étant obtenues par sélection de résistance aux analogues d'acides aminés. De la même manière, cette application est particulièrement intéressante pour augmenter la complexité aromatique de souches de levure capables de surproduire des terpènes, puisque de tels terpènes (géraniol, nérol, beta-citronellol...) se retrouvent ensuite sous une forme acétylée qui est aromatiquement active.

### REFERENCES

Boeke JD., Lacroute F. et Fink GR. Mol. Gen. Gen. (1984) 197 p 345-346.
Bonneaud N, Ozier-Kalogeropoulos O, Li GY, Labouesse M, Minvielle-Sebastia L, Lacroute F. Yeast. 1991 7:609-15. A family of low and high copy replicative, integrative and single-stranded S. cerevisiae/E. coli shuttle vectors.
Casalone E, Fia G, Barberio C, Cavalieri D, Turbanti L, Polsinelli M. Res Microbiol. 1997 148:613-23. Genetic and biochemical characterization of Saccharomyces cerevisiae mutants resistant to trifluoroleucine.
Cauet G, Degryse E, Ledoux C, Spagnoli R, Achstetter T. Eur J Biochem. 1999 261:317-24. Pregnenolone esterification in Saccharomyces cerevisiae. A potential detoxification mechanism.
Cavalieri D, Casalone E, Bendoni B, Fia G, Polsinelli M, Barberio C. Mol Gen Genet. 1999 261:152-60. Trifluoroleucine resistance and regulation of alpha-isopropyl malate synthase in Saccharomyces cerevisiae.
Fujii T, Nagasawa N, Iwamatsu A, Bogaki T, Tamai Y, Hamachi M. Appl Environ Microbiol. 1994 60:2786-92. Molecular cloning, sequence analysis, and expression of the yeast alcohol acetyltransferase gene.
Fujii T, Yoshimoto H, Nagasawa N, Bogaki T, Tamai Y, Hamachi M. Yeast. 1996 12:593-8. Nucleotide sequences of alcohol acetyltransferase genes from lager brewing yeast, Saccharomyces carlsbergensis.
Fujii T, Kobayashi O, Yoshimoto H, Furukawa S, Tamai Y. Appl Environ Microbiol. 1997 63:910-5. Effect of aeration and unsaturated fatty acids on expression of the Saccharomyces cerevisiae alcohol acetyltransferase gene.
Fujiwara D, Yoshimoto H, Sone H, Harashima S, Tamai Y. Yeast. 1998 14:711-21. Transcriptional co-regulation of Saccharomyces cerevisiae alcohol acetyltransferase gene, ATF1 and delta-9 fatty acid desaturase gene, OLE1 by unsaturated fatty acids.
Fujiwara D, Kobayashi O, Yoshimoto H, Harashima S, Tamai Y. Yeast. 1999 15:1183-97. Molecular mechanism of the multiple regulation of the Saccharomyces cerevisiae ATF1 gene encoding alcohol acetyltransferase.
Fukuda K, Watanabe M, Asano K, Ouchi K, Takasawa S. Curr Genet. 1991 20:453-6. A mutated ARO4 gene for feedback-resistant DAHP synthase which causes both o-fluoro-DL-phenylalanine resistance and beta-phenethyl-alcohol overproduction in Saccharomyces cerevisiae.
Fukuda K, Watanabe M, Asano K, Ouchi K, Takasawa S. Curr Genet. 1991 20:449-52. Isolation and genetic study of p-fluoro-DL-phenylalanine-resistant mutants overproducing beta-phenethyl-alcohol in Saccharomyces cerevisiae.
Fukuda K, Yamamoto N, Kiyokawa Y, Yanagiuchi T, Wakai Y, Kitamoto K, Inoue Y, Kimura A. Appl Environ Microbiol. 1998 64:4076-8. Balance of activities of alcohol acetyltransferase and esterase in Saccharomyces cerevisiae is important for production of isoamyl acetate.
Gietz D, St Jean A, Woods RA, Schiestl RH. Nucleic Acids Res. 1992 20:1425. Improved method for high efficiency transformation of intact yeast cells.
Gietz RD, Schiestl RH, Willems AR, Woods RA.Yeast. 1995 11:355-60. Studies on the transformation of intact yeast cells by the LiAc/SS-DNA/PEG procedure.
Ito H, Fukuda Y, Murata K, Kimura A. J Bacteriol. 1983 153:163-8. Transformation of intact yeast cells treated with alkali cations.
Javelot C., Girard P., Colonna-Ceccaldi B. et Vladescu B. 1991 J. Biotechnol. 21 p 239-252.
Lilly M, Lambrechts MG, Pretorius IS. Appl Environ Microbiol. 2000 66:744-53. Effect of increased yeast alcohol acetyltransferase activity on flavor profiles of wine and distillates.
Malcorps P, Dufour JP. Eur J Biochem. 1992 210:1015-22. Short-chain and medium-chain aliphatic-ester synthesis in Saccharomyces cerevisiae.
Nacken V, Achstetter T, Degryse E. Gene. 1996 175:253-60. Probing the limits of expression levels by varying promoter strength and plasmid copy number in Saccharomyces cerevisiae.
Nagasawa N, Bogaki T, Iwamatsu A, Hamachi M, Kumagai C. Biosci Biotechnol Biochem. 1998 62:1852-7. Cloning and nucleotide sequence of the alcohol acetyltransferase II gene (ATF2) from Saccharomyces cerevisiae Kyokai No. 7.
Sherman F. Methods Enzymol. 1991;194:3-21. Getting started with yeast.
Wach A, Brachat A, Pohlmann R, Philippsen P. Yeast. 1994 Dec;10(13):1793-808. New heterologous modules for classical or PCR-based gene disruptions in Saccharomyces cerevisiae.
Walker M. G. J. Am. Soc. Brew. Chem. 1998 56:109-113 Magnesium as a Stress-Protectant for Industrial Strains of *Saccharomyces cerevisiae.*

## Revendications

1. Utilisation d'une composition de microorganismes transformés par le gène *ATF2,* de manière à préparer une boisson fermentée enrichie en esters acétylés apportant une note fruitée souhaitée, sans pour autant augmenter la teneur en esters indésirables d'un point de vue sensoriel au delà de leur seuil de perception et notamment en acétate d'éthyle.

2. Utilisation selon la revendication 1 **caractérisée en ce que** la concentration de la boisson fermentée en esters acétylés indésirables d'un point de vue sensoriel - plus spécialement en acétate d'éthyle - est inférieure à 150mg/l, et typiquement de 10 à 50 mg/l.

3. Utilisation selon la revendication 1 **caractérisée en ce que** la concentration en acétate d'éthyle de la boisson fermentée est de l'ordre de 3 à 30 fois inférieure à celle obtenue avec une composition de microorganismes transformés par le gène *ATF1* et reste en dessous du seuil de perception.

4. Utilisation selon la revendication 1 ou 2 **caractérisée en ce que** la concentration en acétate d'éthyle de la boisson fermentée est de l'ordre de 1 à 10 fois supérieure à celle obtenue avec une composition contrôle de microorganismes non transformés, et reste en dessous du seuil de perception.

5. Utilisation selon l'une quelconque des revendications 1 à 3 **caractérisée en ce que** la concentration de la boisson fermentée en acétate de 2-phényléthyle est de l'ordre de 7 à 70 fois supérieure à celle obtenue avec la composition contrôle, et 1.1 à 4 fois inférieure à celle obtenue avec la composition transformée par *ATF1*.

6. Utilisation selon l'une quelconque des revendications 1 à 4 **caractérisée en ce que** la concentration de la boisson fermentée en acétate d'isoamyle est de l'ordre de 4 à 30 fois supérieure à celle obtenue avec la composition contrôle, et 2 à 10 fois inférieure à celle obtenue avec la composition transformée par *ATF1.*

7. Utilisation selon l'une quelconque des revendications 1 à 5 **caractérisée en ce que** le rapport acétate d'éthyle /acétate de 2-phényléthyle de la boisson fermentée est de l'ordre de 1.4 à 10 fois inférieur à celui d'une boisson fermentée obtenue en utilisant une composition de microorganismes transformés par le gène *ATF1.*

8. Utilisation selon l'une quelconque des revendications 1 à 6 pour la préparation de vin, de bière, de saké, de cidre.

9. Procédé de préparation d'une boisson fermentée enrichie en esters acétylés comprenant les étapes :
- préparation d'une base fermentaire comprenant au moins un précurseur métabolique d'ester acétylé,
- préparation d'une composition de microorganismes transformés par *ATF2,*
- addition de la composition de microorganismes transformés à la base fermentaire, les précurseurs étant choisis parmi les alcools supérieurs, les alcools primaires, les alcools secondaires, les diols, les acides aminés précurseurs des alcools correspondants, les terpènes ou tout autre substrat pour ATF2p, la concentration d'esters acétylés indésirables, plus spécialement d'acétate d'éthyle, dans la boisson étant de l'ordre de 3 à 30 fois inférieure à celle obtenue avec une composition de microorganismes transformés par le gène *ATF1*, typiquement de l'ordre de 10 à 150 mg/l, et de préférence inférieure à 50 mg/l.

10. Procédé selon la revendication 9 **caractérisé en ce que** les alcools sont choisis parmi le n-propanol, le n-butanol, le n-hexanol, le n-heptanol, le n-octanol, le n-décanol, l'isobutanol, le 2-phényléthanol, le benzylalcool, le glycérol.

11. Procédé selon la revendication 9 **caractérisé en ce que** les terpènes sont choisis parmi le géraniol, le citronellol, le nérol.

12. Procédé selon l'une quelconque des revendications 9 à 11 **caractérisé en ce que** les microorganismes sont transformés par une cassette d'expression contenant le gène *ATF2* et au moins une séquence régulatrice de son expression.

13. Procédé selon la revendication 12 **caractérisé en ce que** la cassette d'expression est intégrée dans le génome des microorganismes à l'aide d'un vecteur intégratif

14. Procédé selon la revendication 13 **caractérisé en ce que** la cassette d'expression est introduite dans un vecteur épisomal.

15. Procédé selon l'une quelconque des revendications 9 à 14 **caractérisé en ce que** les microrganismes sont des levures choisies parmi *Saccharomyces cerevisiae, Saccharomyces carlsbergensis.*

16. Procédé selon l'une quelconque des revendications 9 à 15 **caractérisé en ce que** les microorganismes sont des levures mutantes surproductrices de précurseurs métaboliques des esters acétylés souhaités.

17. Procédé selon l'une quelconque des revendications 9 à 16 **caractérisé en ce qu'**il utilise une souche produisant naturellement des terpènes, capable de produire au moins du géraniol et/ou de l'acétyl géraniol.

18. Procédé de préparation d'une boisson à partir d'une boisson fermentée selon l'une quelconque des revendications 9 à 17, la boisson fermentée étant distillée et/ou servant dans un mélange de boissons fermentées ou non fermentées.

19. Boisson susceptible d'être obtenue selon un procédé selon l'une quelconque des revendications 9 à 18 ou selon l'utilisation selon l'une quelconque des revendications 1 à 8.

20. Préparation de microorganismes transformés par le gène *ATF2*, plus spécialement de levures, du type surnageant de culture ou analogue, destinée à la préparation d'une boisson selon la revendication 19.
